# EUROPEAN PATENT SPECIFICATION

(11) **EP 4 059 436 B1**
(45) Date of publication and mention of the grant of the patent: **01.11.2023**
(21) Application number: 20905177.0
(22) Date of filing: 11.12.2020
(51) Int. Cl.: A61B 8/00, A61B 18/00, A61N 7/00, A61B 17/00, A61N 7/02

(54) **ULTRASONIC RADIATION TOOL AND ULTRASONIC DEVICE**
ULTRASCHALLSTRAHLUNGSWERKZEUG UND ULTRASCHALLGERÄT
OUTIL À RAYONNEMENT ULTRASONORE ET DISPOSITIF ULTRASONORE

(30) Priority: 24.12.2019 JP 2019232601
(43) Date of publication of application: 21.09.2022
(73) Proprietor: Kyocera Corporation, Kyoto-shi Kyoto 612-8501 (JP)
(72) Inventor: SATO, Izuru, Kyoto-shi, Kyoto 612-8501 (JP); TOGO, Makoto, Kyoto-shi, Kyoto 612-8501 (JP)
(74) Representative: Viering, Jentschura & Partner mbB Patent- und Rechtsanwälte
(86) International application number: PCT/JP2020/046296
(87) International publication number: WO 2021/131798

(56) References cited:
- JP-A- H06 319 746
- JP-A- 2013 509 932
- JP-A- 2013 543 422
- US-A1- 2009 230 822
- US-A1- 2013 012 841
- US-A1- 2014 316 269
- SON KEON-HO ET AL: "Flat HIFU transducer with a sawtooth-shaped ultrasound radiation face", JOURNAL OF THE KOREAN PHYSICAL SOCIETY, THE KOREAN PHYSICAL SOCIETY, SEOUL, vol. 63, no. 8, 5 November 2013 (2013-11-05), pages 1566-1575, XP035351419, ISSN: 0374-4884, DOI: 10.3938/JKPS.63.1566 [retrieved on 2013-11-05]

## Description

### Technical Field

The present disclosure relates to an ultrasonic radiator that emits ultrasonic waves to a subject, such as a human body. The present disclosure also relates to an ultrasonic device including the ultrasonic radiator.

### Background Art

Ultrasonic devices that emit ultrasonic waves to subjects such as human bodies are known (see, for example, PTL 1 and PTL 2). For example, such an ultrasonic device is used as an ultrasonic therapeutic device for emitting ultrasonic waves to a lesion in a patient under medical treatment or as an ultrasound diagnostic device for capturing a two-dimensional cross-sectional image of a lesion in a patient. Examples of known ultrasonic therapeutic devices include ultrasonic devices intended for high-intensity focused ultrasound (HIFU) therapy. Such an ultrasonic device may have a concave surface from which ultrasonic waves are emitted in such a way as to be focused onto a predetermined location. According to PTL 1 and PTL 2, vibratory elements that vibrate to generate ultrasonic waves are arranged along such a concave surface.
US 2013/012841 A1 discloses a curved high intensity focused ultra-sound (HIFU) transducer comprising a plurality of curved composite ceramic piezoelectric tiles having opposite convex and concave surfaces, each tile having electrodes on the surfaces electrically coupled to the composite ceramic piezoelectric material, and a plurality of acoustic transmission areas located on each tile and actuated through electrodes on the convex surface.
US 2009/230822 A1 discloses a transducer array for lysing an adipose tissue, the transducer array comprising at least one unitary piece of piezoelectric material having first and second opposing surfaces; and one or more conductive layers on each of said first and second opposing surfaces, wherein at least one of said one or more conductive layers comprises a plurality of electrode elements.

### Citation List

### Patent Literature

PTL 1: Japanese Unexamined Patent Application Publication No. 6-261908
PTL 2: Japanese Unexamined Patent Application Publication (Translation of PCT Application) No. 2015-516233

### Summary of Invention

The present invention provides an ultrasonic radiator according to claim 1 and an ultrasonic device according to claim 13.

Preferred embodiments are described in the dependent claims.

### Brief Description of Drawings

[Fig. 1] Fig. 1 schematically illustrates the configuration of an ultrasonic device according to a first embodiment.
[Fig. 2] Fig. 2 is a perspective view of an ultrasonic wave generation part of the ultrasonic device in Fig. 1, illustrating the schematic configuration of the ultrasonic wave generation part.
[Fig. 3] Fig. 3 is a perspective view of a plate-like element included in the ultrasonic wave generation part in Fig. 2.
[Fig. 4] Fig. 4 is a sectional view of the plate-like element taken along line IV-IV in Fig. 3.
[Fig. 5] Fig. 5 is a perspective view of part of an outer surface of a supporter included in the ultrasonic wave generation part in Fig. 2.
[Fig. 6] Fig. 6 is a sectional view of a structure where the plate-like element in Fig. 3 is fixed.
[Fig. 7] Figs. 7(a) and 7(b) schematically illustrate examples of how ultrasonic waves are focused.
[Fig. 8] Fig. 8 is a sectional view of a structure where a plate-like element according to a second embodiment is fixed.
[Fig. 9] Fig. 9 is a sectional view of a structure where a plate-like element according to a third embodiment is fixed.
[Fig. 10] Fig. 10 is a sectional view of a structure where a plate-like element according to a fourth embodiment is fixed.
[Fig. 11] Fig. 11 is a sectional view of a structure where a plate-like element according to a fifth embodiment is fixed.
[Fig. 12] Fig. 12 is a sectional view of a structure where a plate-like element according to a sixth embodiment is fixed.

### Description of Embodiments

Hereinafter, embodiments of the present disclosure will be described with reference to the accompanying drawings. The accompanying drawings are schematic representations. That is, not every detail may be illustrated in the drawings. Constituent elements are not drawn to scale, and the dimension ratios thereof do not fully correspond to the actual dimension ratios. The relative dimensions and the scale ratio may vary from drawing to drawing. For the purpose of emphasizing a particular shape, the outline of the shape may be illustrated in such a manner that a specific dimension looks greater than it really is.

Embodiments that follow a first embodiment will be essentially described with a focus on their distinctive features only. Unless otherwise noted, these embodiments may be equated with the previously described embodiment or may be understood by analogy to the previously described embodiment.

### First Embodiment

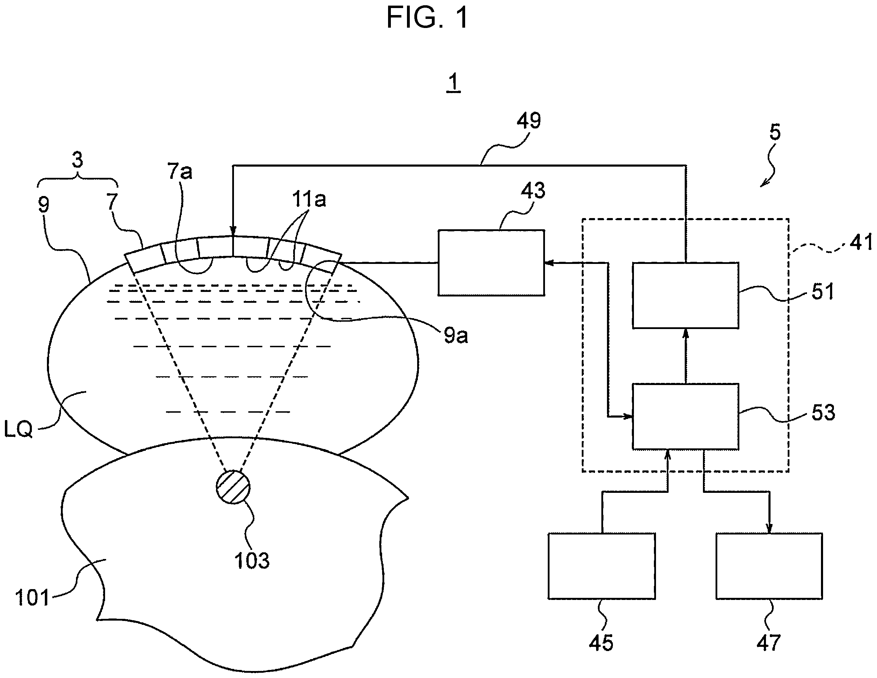

Fig. 1 schematically illustrates the configuration of an ultrasonic device 1 according to a first embodiment.

The ultrasonic device 1 may, for example, be intended for high-intensity focused ultrasound (HIFU) therapy. For example, the ultrasonic device 1 focuses ultrasonic waves onto a lesion 103 in a patient 101. Alternatively, ultrasonic waves may be focused onto foreign matter, such as a calculus. The same applies to the following. This process is accompanied by, for example, the release of heat, which in turn causes denaturation in the lesion 103. The ultrasonic device 1 may be adapted to treatment of various parts of a human body, and the ultrasonic device 1 may be adapted to treatment of various kinds of diseases. In other words, the ultrasonic device 1 may be designed to emit ultrasonic waves of any desired frequency and of any desired intensity, and components of the ultrasonic device 1 each may have desired dimensions. Sound waves with a frequency of 20 kHz or higher are typically referred to as ultrasonic waves. Generally, there is no upper limit frequency for ultrasonic waves; nevertheless, ultrasonic waves may be deemed to cover a frequency range from 20 kHz to 5 GHz.

The ultrasonic device 1 is placed in the immediate area of the patient 101. The ultrasonic device 1 includes an ultrasonic radiator 3 (hereinafter also simply referred to as "radiator 3") and a device main body 5. The ultrasonic radiator 3 is directly involved in ultrasonic radiation. The device main body 5 supplies the radiator 3 with power.

### Ultrasonic Radiator

The radiator 3 includes a generation part 7 and a bag 9. The generation part 7 generates ultrasonic waves. The bag 9 is disposed between the generation part 7 and the patient 101. The generation part 7 has a concave surface 7a, from which ultrasonic waves are emitted. The concave surface 7a is oriented toward the patient 101. The concave surface 7a is substantially in the form obtained by cutting part of a spherical surface (an inner surface of a sphere). Thus, ultrasonic waves emitted from the concave surface 7a are focused onto a region around the center of the sphere. When seen from another perspective, ultrasonic waves are focused onto the lesion 103. A liquid LQ is sealed in the bag 9, at least while the ultrasonic device 1 in operation. The liquid LQ helps curb abrupt variations in the acoustic impedance between the concave surface 7a and the surface of the body of the patient 101.

For example, the liquid LQ is water. Alternatively, the liquid LQ may be a mixture of water and a desired additive. Such an additive may be added to adjust the acoustic impedance. The acoustic impedance of the liquid LQ may be greater than or equal to 1×10⁶ kg/(m²·s) and less than or equal to 2×10⁶ kg/ (m²·s) or may be greater than or equal to 1.3×10⁶ kg/ (m²·s) and less than or equal to 1.7×10⁶ kg/(m²·s). For reference, the following are the acoustic impedances of various kinds of substances: (i) the acoustic impedance of water is about 1.5×10⁶ kg/ (m²·s); (ii) the acoustic impedance of air is about 0; (iii) the acoustic impedance of fat is about 1.4×10⁶ kg/ (m²·s) ; and (iv) the acoustic impedance of muscle is about 1.7×10⁶ kg/(m²·s).

### Generation Part

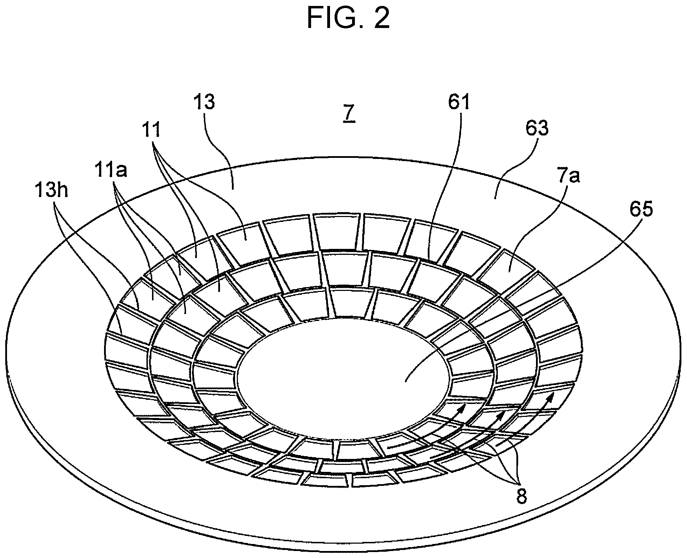

Fig. 2 is a schematic diagram for explanation of the principal structure of the generation part 7. The upper side in Fig. 2 is where the patient 101 stands, sits, or lies. That is, Fig. 2 is a perspective view of the generation part 7, illustrating the generation part 7 viewed from the side on which the concave surface 7a is located.

The generation part 7 includes mainly plate-like elements 11 and a supporter 13. The plate-like elements 11 emit ultrasonic waves. The supporter 13 holds the plate-like elements 11. The supporter 13 is in the form of a frame or, more specifically, has a skeletal structure. Peripheries of the plate-like elements 11 are held by the supporter 13 in such a manner that the plate-like elements 11 are exposed facing the patient 101. The generation part 7 may also include a housing that has any desired shape and covers the illustrated structure from the side opposite to the patient 101.

### Layout of Plate-Like Elements

The plate-like elements 11 each may be substantially in the form of a flat plate. Each plate has a front surface and a back surface, that is, a pair of surfaces that are larger than the other surfaces of the plate. The front surface or the back surface is a radiation surface 11a, from which ultrasonic waves are emitted to the patient 101. The plate-like elements 11 are arranged side by side and inclined at different angles (i.e., laid in different directional orientations) in such a manner that the respective radiation surfaces 11a are directed toward one focal region (i.e., the lesion 103). The plate-like elements 11 constitute the concave surface 7a. That is, the concave surface 7a is composed of flat surfaces (the radiation surfaces 11a); that is, the concave surface 7a is not a single curved surface.

The plate-like elements 11 may be arranged in any desired pattern. The plate-like elements 11 in the illustrated example are arranged in the circumferential direction of the concave surface 7a (i.e., along the periphery of the concave surface 7a) in such a way as to form several element alignments 8 (marked with the respective arrows). The element alignments 8 are each in the form of a ring. As in the illustrated example, two or more element alignments 8 may be arranged in the form of multiple (i.e., two or more) rings (e.g., arranged concentrically) when the concave surface 7a is viewed in plan. Alternatively, only one element alignment 8 may be provided. The center-to-center distance may be constant throughout each element alignment 8 as in the illustrated example or may be irregular, where the center-to-center distance is the distance between the center of one plate-like element 11 and the center of another plate-like element 11 adjacent thereto (e.g., the distance between the geometric centers of two adjacent plate-like elements 11 viewed in plan). Any desired number of plate-like elements 11 may be included in each element alignment 8. Two or more element alignments 8 may include the same number of plate-like elements 11. In some embodiments, the number of plate-like elements 11 included in one element alignment 8 is not equal to the number of plate-like elements 11 included in another element alignment 8.

The plate-like elements 11 are not disposed in an innermost region of the concave surface 7a in the illustrated example. The inner most region is a midsection 65, which will be described later. Any desired electronic component may be disposed in this region. For example, an ultrasonic sensor for determining the position of the lesion 103, a visual sensor for determining the position of a marker placed on the surface of the body of the patient 101 and showing the location of the lesion 103, and/or a receiving unit for receiving reflected waves of ultrasonic waves emitted from the plate-like elements 11 may be disposed in the region concerned. The innermost region of the concave surface 7a may have an opening. For example, visible light detected by the visual sensor and/or ultrasonic waves detected by the ultrasonic sensor may be allowed to pass through the opening. In some embodiments, the innermost region of the concave surface 7a is a mounting place for the plate-like elements 11.

### Shape of Plate-Like Elements Viewed in Plan

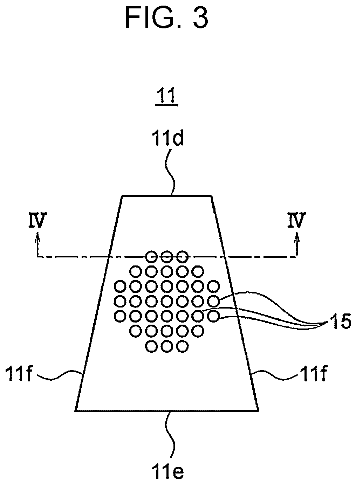

Fig. 3 is a plan view of one of the plate-like elements 11.

The plate-like elements 11 each may have any desired shape and desired dimensions when viewed in plan. For example, the plate-like elements 11 may be geometrically and dimensionally identical to each other. Alternatively, two or more kinds of plate-like elements 11 may be provided, where these plate-like elements 11 are geometrically and/or dimensionally different from each other. When viewed in plan, the plate-like elements 11 may be shaped (like segments into which a spherical surface is divided) such that the plate-like elements 11 are adjacent to each other with a relatively small gap therebetween, as in the example illustrated in Figs. 2 and 3. Alternatively, the plate-like elements 11 may, for example, each be circular in shape when viewed in plan.

When viewed in plan, the plate-like elements 11 illustrated in Figs. 2 and 3 each have a trapezoidal shape, in which case the plate-like elements 11 are adjacent to each other with a relatively small gap therebetween. It is obvious that the plate-like elements 11 each having a polygonal shape other than the trapezoidal shape can be arranged with a small gap therebetween to constitute the concave surface 7a, which may have the shape of a regular polyhedron (a Platonic solid), a semi-regular Polyhedron (an Archimedean solid), and a Platonic solid and may also be shape like a dome as employed in various technical fields.

More specifically, the plate-like elements 11 each have the shape of an isosceles trapezoid that has two bases and a pair of legs. The upper base, which is denoted by 11d in Fig. 3, is closer than the lower base to the inside of the concave surface 7a. The lower base, which is denoted by 11e in Fig. 3, is closer than the upper base to the periphery of the concave surface 7a. The leges, which are denoted by 11f in Fig. 3, are of equal length. The trapezoidal shape of each plate-like element 11 may have desired dimensions, and the length of the upper base 11d, the length of the lower base 11e, and the height of the trapezoid (the distance between the upper base 11d and the lower base 11e) each may be greater than the others.

As mentioned above, the plate-like elements 11 are arranged in the circumferential direction of the concave surface 7a to provide the element alignments 8 that are each in the form of a ring. The plate-like elements in each element alignment 8 are arranged in such a manner that one of the legs 11f of one plate-like element 11 is adjacent to one of the legs 11f of another plate-like element 11. The two adjacent plate-like elements 11 may be arranged in such a manner that one of the legs 11f of one plate-like element 11 is or is not parallel to one of the legs 11f of the other plate-like elements 11. In the latter case, the distance between these two legs 11f may increase with increasing proximity to the inside of the concave surface 7a or with increasing proximity to the periphery of the concave surface 7a. Two adjacent element alignments 8 are arranged in such a manner that the upper base 11d of the trapezoidal shape of each of the plate-like elements 11 in one element alignment 8 is adjacent to the lower base 11e of the trapezoidal shape of the corresponding one of the plate-like elements 11 in the other element alignment 8.

The plate-like elements 11 each have four lateral surfaces 11s, which will be described later. Each of the four lateral surfaces 11s (or a lateral surface 19s or 21s included in the lateral surface 11s) may be hereinafter also referred to as the upper base 11d, the lower base 11e, and the pair of legs 11f.

### Structure of Plate-Like Elements

Referring to Fig. 3, the plate-like elements 11 each include vibratory elements 15 or, more specifically, piezoelectric elements. The vibratory elements 15 vibrate to generate ultrasonic waves. Any desired number of vibratory elements 15 may be included. The vibratory elements 15 each may be place in any desired location. The vibratory elements 15 each may have any desired planar shape and desired dimensions.

The vibratory elements 15 in the illustrated example are substantially evenly distributed in the planar direction of the plate-like elements 11. The term "planar direction" herein refers to the direction parallel to the subject viewed in plan. More specifically, the vibratory elements 15 are arranged in rows and columns in such a manner that the center-to-center distance is constant, where the center-to-center distance is the distance between the center of one vibratory element 15 and the center of another vibratory element 15 adjacent thereto (e.g., the distance between the geometric centers of two adjacent vibratory elements 15 viewed in plan). The vibratory elements 15 may be arranged in such a manner that the position of the center of each vibratory element 15 in one column and the position of the center of the corresponding vibratory element 15 in the adjacent column do not coincide with each other in any direction, with the amount of misalignment in one direction being equivalent to about half the center-to-center distance. The vibratory elements 15 may be arranged along concentric circles or may be arranged radially. It is not required that the vibratory elements 15 be evenly distributed. The arrangement direction of the vibratory elements 15 may bear any desired relationship to the direction in which peripheral portions of the plate-like elements 11 extend.

The area of the region in which the vibratory elements 15 are placed (e.g., the area of a minimum convex polygon in which the vibratory elements 15 fit) may be greater than or equal to 1/5, 1/2, 2/3, or 4/5 of the area of each plate-like element 11 (or the area of the portion that is not covered by the supporter 13 and is exposed facing the patient 101). In the case where the area of the region concerned is greater than or equal to 4/5 of the area of each plate-like element 11, it can be construed that the vibratory elements 15 are distributed over the entire surface area of the plate-like element 11. In the case where the vibratory elements 15 are not distributed over the entire surface area of the plate-like element 11, the vibratory elements 15 may be located in any desired region, which may be close to the center or the periphery of the plate-like element 11. The region concerned may have any desired shape.

The vibratory elements 15 in the illustrated example are circular when viewed in plan. When seen from another perspective, the shape of each vibratory element 15 viewed in plan has line symmetry or rotational symmetry. Alternatively, each vibratory element 15 viewed in plan may have any other shape that is, for example, elliptical, polygonal, or asymmetrical. In a case where the shape of each vibratory element 15 viewed in plan is not circular, each vibratory element 15 viewed in plan may be in any desired directional orientation with respect to the shape of the plate-like element 11 viewed in plan.

### Multilayer Structure of Plate-Like Elements

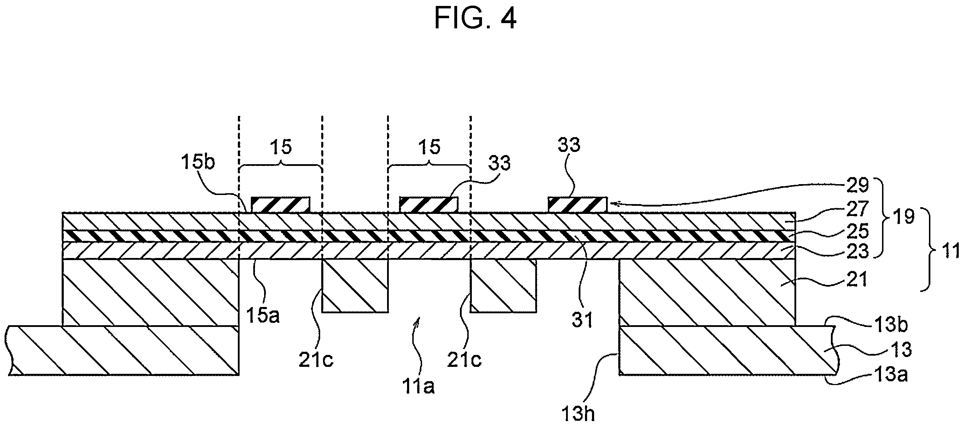

Fig. 4 is a sectional view of the plate-like element taken along line IV-IV in Fig. 3. The lower side in Fig. 4 is where the patient 101 stands, sits, or lies. Fig. 4 illustrates part of the supporter 13 as well as one plate-like element 11.

The plate-like element 11 includes two or more layers extending in the planar direction of the plate-like element 11 (i.e., along the radiation surface 11a). The layers each may be in the form of a plate. More specifically, the plate-like element 11 includes mainly an element substrate 19 and a cavity member 21. The element substrate 19 includes the vibratory elements 15. The cavity member 21 is disposed on the element substrate 19. For example, the plate-like element 11 is disposed in such a manner that the cavity member 21 (or the supporter 13, to be more precise) faces the patient 101. The region corresponding to the vibratory elements 15 is bent and distorted such that the element substrate 19 vibrates. The vibration is transferred to the fluid that is adjacent one side of the element substrate 19 closer than the reverse side of the element substrate 19 to the patient 101, and as a result, ultrasonic waves are generated. The cavity member 21 has cavities 21c (openings, holes), each of which is covered with the corresponding one of the vibratory elements 15 included in the element substrate 19. Peripheral portions of the cavities 21c of the cavity member 21 are helpful in defining fixed ends of the vibratory elements 15. The cavity member 21 thus aids in adjusting the natural frequency (resonant frequency) of the vibratory elements 15.

The cavities 21c and second electrodes 33 (individual electrodes), which will be described later, are provided on the main surfaces (largest surfaces, namely, a front surface and a back surface) of the plate-like element 11; that is, the main surfaces of the plate-like element 11 have projections and recesses and are not flat. With regard to the plate-like element 11, it is suggested that being plate-like in shape or being in the form of a flat plate does not necessarily refers to the shape of a plate or a flat plate in a strict sense. For example, the plate-like element 11 may be mainly composed of flat layers each having a constant thickness. In this respect, it can be construed that the plate-like element 11 is in the form of a flat plate. The layers, which will be described later, are denoted by 23, 25, and 27, respectively. It may be construed that the plate-like element 11 is in the form of a flat plate in a case where tops of the projections (or bottoms of the recesses) in each of the two main surfaces of the plate-like element 11 are located in the same plane. It may also be construed that the plate-like element 11 is in the form of a flat plate in a case where the percentage of the ratio of the arithmetic mean roughness Ra of each main surface with projections and recesses to the equivalent circle diameter of a circle with an area equivalent to the area of the plate-like element 11 is less than or equal to 5%, 2%, or 1%.

### Element Substrate

The element substrate 19 includes two or more members stacked in layers and extending in the planar direction of the element substrate 19 (i.e., along the radiation surface 11a). More specifically, the element substrate 19 includes a vibration layer 23, a first conductor layer 25, a piezoelectric layer 27, and a second conductor layer 29, which are stacked in this order from the side on which the patient 101 stands, sits, or lies. When seen from another perspective, these layers are stacked in this order, with the vibration layer 23 located on the cavity member 21. The first conductor layer 25 includes, for example, a first electrode 31. The second conductor layer 29 includes, for example, the second electrodes 33. The first electrode 31 and the second electrodes 33 are arranged in such a manner that the piezoelectric layer 27 is located between the first electrode 31 and the array of the second electrodes 33. Alternating voltage is applied to a pair of electrodes (the first electrode 31 and the second electrodes 33) such that the region being part of the element substrate 19 and corresponding to the vibratory elements 15 vibrate in a manner so as to be bent and distorted. The element substrate 19 may include, in addition to the layers illustrated in the drawing, any desired layer such as an insulating layer that lies over the second conductor layer 29.

The region being part of the element substrate 19 and regarded as the vibratory elements 15 may be defined as appropriate. For convenience, the vibratory elements 15 in the present embodiment are defined as a region that is part of the element substrate 19 and is located on the cavities 21c (or, more specifically, a region that covers opening faces located on the element substrate 19 and defined by the cavities 21c). Alternatively, the vibratory elements 15 may be defined as a region on the second electrodes 33 (individual electrodes).

The vibratory elements 15 each include a first surface 15a and a second surface 15b. The first surface 15a is oriented toward the cavity 21c (the patient 101). The second surface 15b is oriented opposite the cavity 21c. The first surface 15a may be part of a surface of the vibration layer 23 or, more specifically, a surface located on the cavity member 21. The second surface 15b may be composed of part of a surface of the second conductor layer 29 and part of a surface of the piezoelectric layer 27. More specifically, these surfaces are oriented opposite the cavity member 21, and the relevant part of the surface of the piezoelectric layer 27 is not covered with the second conductor layer 29. Alternatively, an insulating layer may lie over the second conductor layer 29, in which case the second surface 15b may be part of the insulating layer. When the vibratory elements 15 vibrate, ultrasonic waves directed toward the patient 101 are generated in the first surfaces 15a, which constitute part of the radiation surface 11a of the plate-like element 11.

### Vibration Layer

The vibration layer 23 is large enough to extend across the vibratory elements 15 (e.g., substantially all over the element substrate 19), essentially with no gap between one part and another part of the vibration layer 23. The thickness of the vibration layer 23 is substantially constant. The vibration layer 23 restricts distortion of the piezoelectric layer 27 in the planar direction in such a way as to aid in causing out-of-plane vibration.

The vibration layer 23 is made of, for example, an insulating material or a semiconductor material. The material of the vibration layer 23 may be an inorganic material or an organic material. More specifically, the vibration layer 23 and the piezoelectric layer 27 may be made of the same piezoelectric material or may be made of different piezoelectric materials. The material of the piezoelectric layer 27 will be described later. For example, the vibration layer 23 may be made of silicon (Si), silicon dioxide (SiO₂), silicon nitride (SiN), or sapphire (Al₂O₃). Alternatively, the vibration layer 23 may be such that different layers made of different materials are arranged in a stack.

### First Conductor Layer and First Electrode

The first conductor layer 25 in the illustrated example includes the first electrode 31 only. The first electrode 31 may be a common electrode. The common electrode is large enough to extend across the vibratory elements 15 (i.e., substantially all over the element substrate 19), essentially with no gap between one part and another part of the common electrode. The thickness of the first electrode 31 is substantially constant. A through-conductor (not illustrated) extends though the piezoelectric layer 27 to form an electrical connection between the first electrode 31 and wiring (not illustrated) that is disposed opposite the bag 9 with the plate-like element 11 therebetween. The wiring may, for example, be flexible printed circuitry (FPC) 35, which will be described later.

The first conductor layer 25 may be made of any desired metal. For example, the first conductor layer 25 may be made of gold (Au), silver (Ag), palladium (Pd), platinum (Pt), aluminum (Al), nickel (Ni), copper (Cu), chromium (Cr), or an alloy of theses metals. Alternatively, the first conductor layer 25 may be such that different layers made of different materials are arranged in a stack. Still alternatively, the first conductor layer 25 may be made of a material obtained by firing a conductive paste containing one or more of the metals mentioned above. That is, the first conductor layer 25 may be made of a material mixed with an additive such as glass powder and/or ceramic powder (an inorganic insulating material).

### Piezoelectric Layer

The piezoelectric layer 27 is large enough to extend across the vibratory elements 15 (e.g., substantially all over the element substrate 19), essentially with no gap between one part and another part of the piezoelectric layer 27. The thickness of the piezoelectric layer 27 is substantially constant. Alternatively, the piezoelectric layer 27 may be composed of discrete pieces, in which case each of the discrete pieces is provided for the corresponding one of the vibratory elements 15.

The piezoelectric layer 27 may be made of a single-crystal material, a polycrystalline material, an inorganic material, an organic material, a ferroelectric material, a nonferroelectric material, a pyroelectric material, or a nonpyroelectric material. Examples of the inorganic material include lead zirconate titanate materials and lead-free inorganic piezoelectric materials. The lead-free inorganic piezoelectric materials that can be used as the material of the piezoelectric layer 27 may, for example, be perovskite compound materials. Examples of the organic material include polyvinylidene fluoride (PVDF).

The piezoelectric layer 27 may be made of a piezoelectric ceramic plate (a sintered body) or a piezoelectric ceramic thin film. The piezoelectric ceramic plate is a plate-like organic polycrystalline material including piezoelectric crystal grains (and grain boundaries) and is also known as a piezoelectric ceramic plate. The crystal grains included in the piezoelectric ceramic plate have a relatively small aspect ratio and are isotropically distributed. The piezoelectric thin film is a piezoelectric material, such as an inorganic single crystal material, an inorganic polycrystalline material, or an organic material (polymer), and is in the form of a thin film. The piezoelectric thin film that is a polycrystalline material is typically composed of columnar crystals extending in the thickness direction. The piezoelectric thin film typically has a high degree of orientation of crystals and is thus highly piezoelectric.

In at least part of the piezoelectric layer 27 or, more specifically, in at least the region corresponding to the vibratory elements 15, the polarization axis (also referred to as the electrical axis or the X-axis in a single crystal) is substantially parallel to the thickness direction of the piezoelectric layer 27 (i.e., the direction in which the first electrode 31 faces the second electrodes 33). The other part of the piezoelectric layer 27, that is, a region that does not correspond to the vibratory elements 15 may be polarized or unpolarized. The part corresponding to the vibratory elements 15 and the other part of the piezoelectric layer 27 may be polarized in the same direction or in different directions.

### Second Conductor Layer and Second Electrodes

The second conductor layer 29 may include, in addition to the second electrodes 33, wiring (not illustrated) connected to the second electrodes 33. Extended electrodes (not illustrated) (or the wiring) included in the second conductor layer 29 may form an electrical connection between each second electrode 33 and the wiring (not illustrated) that is disposed opposite the bag 9 with the plate-like element 11 therebetween. The wiring disposed opposite the bag 9 may, for example, be the FPC 35, which will be described later.

The second electrodes 33 are individual electrodes each of which is provided for the corresponding one of the vibratory elements 15. The individual electrodes are geometrically discrete and are not necessarily placed at different potentials. For example, two or more second electrodes 33 (e.g., all of the second electrodes 33 included in one plate-like element 11) may be electrically connected to each other. The connection may be formed by the wiring (not illustrated) included in the second conductor layer 29 or may be formed by another means (e.g., the FPC 35, which will be described later). The second electrodes 33 may be placed at the respective potentials. Alternatively, the second electrodes 33 may be divided into groups, each of which includes two or more second electrodes 33, with different groups of the second electrodes 33 being placed at different potentials.

The second electrodes 33 each may have any desired planar shape and desired dimensions. For example, the second electrodes 33 viewed in plan may be geometrically similar or analogous to the vibratory elements 15 viewed in plan (geometrically similar or analogous to the openings defined by the cavities 21c) or may be geometrically different from them. The second electrodes 33 viewed in plan may be circular, elliptical, or polygonal. The entirety of each of the peripheries of the second electrodes 33 viewed in plan may be located on the inner side with respect to the peripheral portion of the opening defined by the corresponding one of the cavities 21c or may substantially coincide with the peripheral portion concerned. The entirety of each of the peripheries of the second electrodes 33 viewed in plan may be located on the outer side with respect to the peripheral portion concerned. Only part of each of the peripheries of the second electrodes 33 viewed in plan may coincide with the peripheral portion concerned or may be located on the inner side with respect to the peripheral portion concerned. Each of the second electrodes 33 in the present embodiment is circular in shape and is located on the inner side with respect to the peripheral portion of the opening defined by the corresponding one of the cavities 21c, which are circular in shape.

The second conductor layer 29 and the first conductor layer 25 may be made of the same material or may be made of different materials. In either case, the material of the second conductor layer 29 may be understood as analogous to the aforementioned material of the first conductor layer 25.

### Workings of Vibratory Elements

When the first electrode 31 and the second electrodes 33 apply an electric field to the piezoelectric layer 27 located between the first electrode 31 and the array of the second electrodes 33 in such a manner that the direction of application of the electric field coincides with the polarization direction, the piezoelectric layer 27 contracts in the planar direction. This contraction is restricted by the vibration layer 23 such that the vibratory elements 15 are bent (undergoes displacement) toward the vibration layer 23 just like a bimetal does. When an electric field is applied opposite to the polarization direction, the vibratory elements 15 are bent toward the piezoelectric layer 27.

The displacement of the vibratory elements 15 induces pressure waves in a medium (e.g., a fluid) around the vibratory elements 15. Then, an electrical signal (a driving signal) with the voltage varying in a predetermined waveform is input to the first electrode 31 and the second electrodes 33 such that ultrasonic waves reflective of the waveform (the frequency and the amplitude) of the electrical signal are generated.

As mentioned above, the vibratory elements 15 vibrate in a manner so as to be bent and distorted. The vibration of each vibratory element 15 is regarded as out-of-plane vibration (bending and vibration) in the primary mode, in which the midsection of the vibratory element 15 viewed in plan is a vibration antinode, and the periphery of the vibratory element 15 viewed in plan (e.g., a region close to the peripheral portion of the cavity 21c) is a vibration node. For example, the resonant frequency of the vibratory elements 15 vibrating in this manner is in the frequency range of ultrasonic waves. The resonant frequency may be set by selecting the materials of the layers constituting the vibratory elements 15, that is, by adjusting the Young's modulus and the density and by adjusting the diameter of the vibratory elements 15 and the thicknesses of the layers, that is, by adjusting the mass and the flexural rigidity of the layers. The resonant frequency may be set with consideration given to the influence of the fluid around the vibratory elements 15 and to the rigidity of the portion (e.g., the cavity member 21) that supports the vibratory elements 15.

The electrical signal may be such as to alternately apply a voltage for causing displacement of the vibratory elements 15 toward the vibration layer 23 and a voltage for causing displacement of the vibratory elements 15 toward the piezoelectric layer 27. That is, the electrical signal may be such as to reverse the polarity (positive and negative) (such as to repeatedly reverse the direction of voltage (electric field) along the polarization axis of the piezoelectric layer 27). Alternatively, the electrical signal may be such as to repeatedly apply only a voltage for causing displacement of the vibratory elements 15 toward the vibration layer 23 or may be such as to repeatedly apply only a voltage for causing displacement of the vibratory elements 15 toward the piezoelectric layer 27. The vibratory elements 15 have resilience such that bending and unbending of the vibratory elements 15 alternate to generate ultrasonic waves.

### Cavity Member

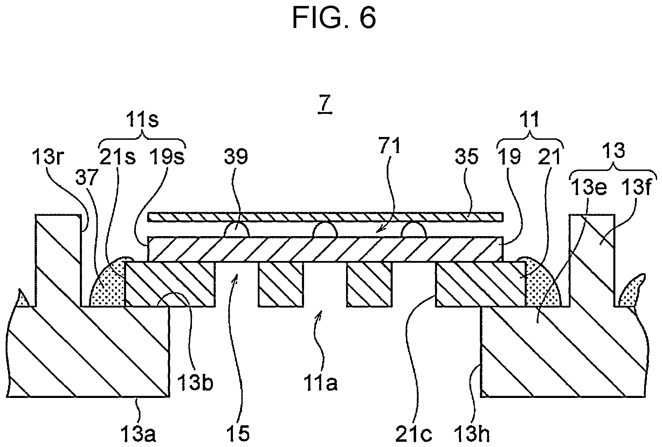

The cavity member 21 is large enough to extend across the vibratory elements 15 and has a constant thickness, where the cavities 21c are left out of consideration. The cavity member 21 and the element substrate 19 may be equal in area (as in the example illustrated in Fig. 4) or may be unequal in area. Another example will be described later with reference to, for example, Fig. 6, in which the cavity member 21 is slightly broader than the element substrate 19. Alternatively, the element substrate 19 may be broader than the cavity member 21.

The cavity member 21 may be made of any desired material, such as an insulating material, a semiconductor material, a conductive material, an inorganic material, an organic material, or a piezoelectric material. The cavity member 21 and one or more layers included in the element substrate 19 may be made of the same material. More specifically, the cavity member 21 may be made of metal, resin, or a ceramic material. The cavity member 21 may be made of two or more materials or may include two or more layers. For example, the cavity member 21 may include a metal layer (including a metal plate) laid on the element substrate 19 and an insulating layer on the metal layer or may be made of glass epoxy resin composed of glass fibers impregnated with epoxy resin.

The cavities 21c each may have any desired shape. In the illustrated example, the shape of each cavity 21c viewed in cross section parallel to the element substrate 19 is constant in the direction in which the cavity 21c extends. Alternatively, the cavities 21c each may have a tapered surface such that each cavity 21c increases or decreases in diameter with increasing proximity to the element substrate 19. The vibratory elements 15 in the present embodiment are defined as a region that is part of the element substrate 19 and is located on the cavities 21c. The shape of the cavities 21c viewed in cross section may thus be understood as analogous to the aforementioned shape of the vibratory elements 15 viewed in plan.

The cavities 21c each may have any desired depth, where the depth refers to the dimension in the direction in which each cavity 21c extends. When seen from another perspective, the cavity member 21 may have any desired thickness. For example, the depth of each cavity 21c may be greater than or equal to 1/20, 1/10, or 1/2 of the diameter of the cavity 21c or may be greater than or equal to the diameter of the cavity 21c. The depth of each cavity 21c may be less than or equal to 10 times or 5 times the diameter of the cavity 21c. The depth of each cavity 21c may be less than or equal to the diameter of the cavity 21c or may be less than or equal to 1/2 or 1/10 of the diameter of the cavity 21c. In a case where the cavities 21c are not circular in cross section, the word "diameter" may be read as "equivalent circle diameter". Any desired combination of these lower and upper limits may be applied unless there is a contradiction between them.

### Supporter

Referring back to Fig. 2, the shape of the supporter 13 is such as to hold the peripheries of the plate-like elements 11. More specifically, the supporter 13 may be geometrically identical to the gaps (boundaries) between adjacent ones of the plate-like elements 11. In other words, the supporter 13 has openings 13h, each of which is covered with the corresponding one of the plate-like elements 11. As illustrated in Fig. 4, the peripheral portion of each plate-like element 11 is laid on a back surface 13b of the supporter 13, that is, on a surface located opposite the patient 101. Through each opening 13h, the radiation surface 11a of the corresponding plate-like element 11 is exposed to view from the side on which the patient 101 stands, sits, or lies.

The supporter 13 is composed of three sections that are substantially concentric when the concave surface 7a is viewed in plan. One is a grating section 61, which is in the form of a grating with the openings 13h. The grating section 61 is strip-shaped and extends annularly over the region in which the plate-like elements 11 are arranged. Another is a rim section 63, which is in the form of a flange and extends outward from the outer periphery of the grating section 61. The other is the midsection 65, which extends from the inner periphery of the grating section 61. These sections hold the peripheries of the plate-like elements 11.

In another example (not illustrated), the midsection 65 may be an opening, and the distance between the inner edge and the outer edge of the rim section 63 (the difference between the outer diameter and the inner diameter) may be reduced. In this case, the supporter 13 may be deemed to include the grating section 61 only. As mentioned above, the region corresponding to the midsection 65 may be a mounting place for the plate-like elements 11; that is, the grating section 61 may stretch to the middle.

The openings 13h each may have any desired shape and each may be of any desired area. The shape of the openings 13h may be similar or analogous to the outer shape of the plate-like elements 11 as in the illustrated example, or the openings 13h may be geometrically different from the plate-like elements 11. For example, the openings 13h may decrease or increase in diameter with increasing proximity to the back surface 13b and with increasing distance from the reverse surface, namely, a front surface 13a, which is closer than the back surface 13b to the patient 101. Alternatively, the shape of the openings 13h may be such that the diameter of each opening 13h is constant. The area of each opening 13h (e.g., the minimum area of each opening 13h that decreases or increases in diameter) may be greater than or equal to 60% of the area of each plate-like element 11 or may be greater than or equal to 80% of the area of each plate-like element 11.

The supporter 13 may be made of any desired material. For example, the supporter 13 may be made of metal, a ceramic material, resin, or a combination of two or more of these. Examples of the metal include stainless steel.

### Details of Shape of Supporter

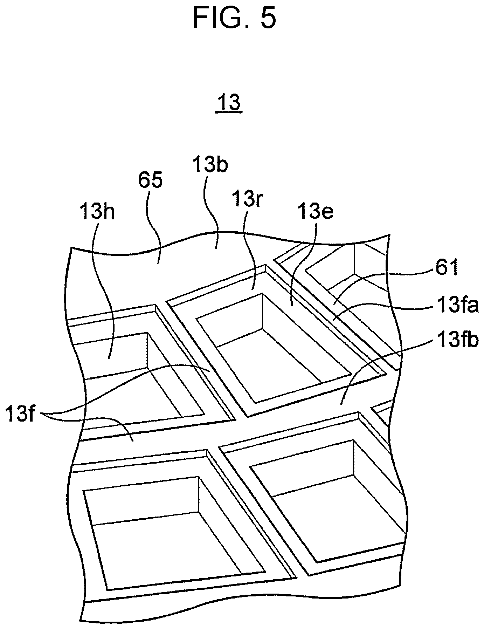

Fig. 5 is a perspective view of part of an example of the back surface 13b of the supporter 13 (i.e., the surface located opposite the lesion 103). Fig. 5 illustrates a state in which the supporter 13 is yet to be fitted with the plate-like elements 11.

The surface that is a mounting place for the plate-like elements 11 (the back surface 13b of the supporter 13 in the illustrated example) may have recesses 13r, which are provided to accommodate the plate-like elements 11. When seen from another perspective, the supporter 13 includes an overlapping portion 13e and a partition portion 13f. The overlapping portion 13e overlaps the plate-like elements 11 in the thickness direction of the plate-like element 11. In the present embodiment, the overlapping portion 13e overlaps the peripheral portions of the plate-like elements 11. The partition portion 13f protrudes from the overlapping portion 13e to the side on which the plate-like elements 11 are to be disposed. In some embodiments, the supporter 13 does not have the recesses 13r (the partition portion 13f).

The partition portion 13f is located between adjacent ones of the plate-like elements 11. More specifically, the supporter 13 in the illustrated example includes partition portions 13fa and partition portions 13fb. The partition portions 13fa are each located between the plate-like elements 11 that are adjacent to each other in the circumferential direction of the supporter 13. The partition portions 13fb are each located between the plate-like elements 11 that are adjacent to each other in the radial direction of the supporter 13. The partition portions 13fa extend, for example, in the radial direction of the supporter 13. The partition portions 13fb extend, for example, in the circumferential direction of the supporter 13.

Each recess 13r may be shaped and sized to be fitted with the corresponding plate-like element 11 such that the recesses 13r aid in positioning the plate-like element 11. When the recesses 13r are fitted with the plate-like elements 11, there may be a gap (clearance) between a side wall of each plate-like element 11 and a wall surface of the corresponding recess 13r such that each plate-like element 11 can move in its planar direction. The gap may be used for the placement of a first adhesive 37, which will be described later. The size of the gap is such that the periphery of each plate-like element 11 is entirely located outside the corresponding opening 13h, irrespective of any displacement of the plate-like element 11 in the planar direction within the recess 13r. Alternatively, each recess 13r may be greater than the size that is small enough to aid in positioning the plate-like element 11.

The recesses 13r viewed in the thickness direction thereof may be geometrically similar to the plate-like elements 11 viewed in plan and/or to the openings 13h viewed in the opening direction thereof. The shape of the recesses 13r may thus be understood as analogous to the aforementioned shape of the plate-like elements 11 viewed in plan. Each recess 13r may include a portion where the wall surface of the recess 13r comes into contact with the side wall of the corresponding plate-like element 11 in such a way as to effect positioning of the plate-like element 11. The other part of each recess 13r may be located away from the side wall of the plate-like element 11 such that a gap for the placement of the first adhesive 37, which will be described later, is provided. The wall surfaces of the recesses 13r each may be a perpendicular wall. Alternatively, the wall surfaces of the recesses 13r each may be an inclined wall, in which case the recesses 13r decrease or increase in diameter. The depth of the recesses 13r may be less than, equal to, or greater than the thickness of the plate-like elements 11.

The partition portion 13f may be in the shape of constant width and/or constant height or may be in the shape of varying width and/or varying height. The partition portion 13f may extend linearly or may be bent at any desired point. The partition portions 13fa may be geometrically identical to each other or may be geometrically different from each other. The same holds true for the partition portions 13fb.

### Structure Where Plate-Like Elements Are Fixed to Supporter

Fig. 6 is a sectional view of a structure where the plate-like elements 11 are fixed to the supporter 13. Details of the element substrate 19 are not illustrated in Fig. 6, which is a sectional view analogous to Fig. 4. The region illustrated in Fig. 6 is slightly greater than the region illustrated in Fig. 4. The plate-like element 11 illustrated in Fig. 6 has a pair of lateral surfaces 11s, which corresponds to a pair of legs 11f when Fig. 6 is considered as a sectional view of the plate-like element 11 taken along line IV-IV in Fig. 3. The pair of lateral surfaces 11s may be regarded as either combination of two lateral surfaces of the plate-like element 11.

As illustrated in Fig. 6, the plate-like element 11 and the supporter 13 are bonded together with the first adhesive 37, which is applied to the lateral surfaces 11s of the plate-like element 11. More specifically, the lateral surfaces 11s of the plate-like element 11 each include a lateral surface 21s of the cavity member 21 and a lateral surface 19s of the element substrate 19. The first adhesive 37 is applied to the lateral surfaces 21s, not to the lateral surfaces 19s. The first adhesive 37 may be applied not only to the lateral surfaces 21s but also to another surface of the cavity member 21 (e.g., a surface located opposite the supporter 13) as in the illustrated example. Applying the first adhesive 37 to such a surface is not a must.

For the sake of clarity, the lateral surface is defined as a surface that forms a connection between a front surface and a back surface of a plate-like shape (a pair of surfaces that are larger than the other surfaces), or the lateral surface is a surface extending in the thickness direction of the plate-like shape. The plate-like elements 11 in the present embodiment each have, for example, a trapezoidal shape, in which case the plate-like elements 11 each have four lateral surfaces 11s. In a case where the plate-like elements 11 are circular in shape, it may be considered that the plate-like elements 11 each have one lateral surface 11s. Alternatively, the lateral surface 11s may be divided as appropriate; that is, it may be considered that the plate-like elements 11 each have two lateral surfaces 11s on both sides of the radiation surface 11a, with one lateral surface 11s on one side of the radiation surface 11a and the other lateral surface 11s on the opposite side of the radiation surface 11a.

In the thickness direction of the plate-like element 11, the first adhesive 37 may be applied to the entirety of the lateral surface 21s of the cavity member 21 as in the illustrated example or may be applied to only part of the lateral surface 21s. In the latter case, some discretion is allowed as to the proportion of the region coated with the first adhesive 37 in the total area of the lateral surface 21s. For example, the region extending in the thickness direction of the plate-like element 11 and coated with the first adhesive 37 may be more than or equal to one half of the lateral surface 21s or may be less than one half of the lateral surface 21s. In the case where the first adhesive 37 is applied to only part of the lateral surface 21s, the region being part of the lateral surface 21s and coated with the first adhesive 37 may be located opposite the element substrate 19 or may be on or close to the element substrate 19. When the overlapping portion 13e, the cavity member 21, and the element substrate 19 are stacked in this order as in the illustrated example, the first adhesive 37 may be used in lesser amounts such that the first adhesive 37 is applied to only part of the lateral surface 21s that is closer than the other part of the lateral surface 21s to the overlapping portion 13e. Alternatively, air may be put into the first adhesive 37 on the overlapping portion 13e such that the first adhesive 37 is applied to only part of the lateral surface 21s that is closer than the other part of the lateral surface 21s to the element substrate 19.

The first adhesive 37 may be applied to the lateral surfaces 11s (or, more specifically, the lateral surfaces 21s in the present embodiment) in a manner so as extend along the entire periphery of the plate-like element 11 (i.e., along the upper base 11d, the lower base 11e, and a pair of legs 11f) or in a manner so as to extend along only part of the periphery of the plate-like element 11. In the latter case, the first adhesive 37 may be applied to only part of the periphery of the plate-like element 11, with a gap being left between each lateral surface 21s and the wall surface of the recess 13r and extending along the entire periphery of the plate-like element 11. Alternatively, the first adhesive 37 may be applied to only part of the periphery of the plate-like element 11, with part of the wall surface of the recess 13r being in direct contact with the lateral surface 21s.

The first adhesive 37 may be applied to the lateral surfaces 11s located on both sides with the radiation surface 11a (or, more specifically, at least one vibratory element 15) therebetween. In the case where the plate-like element 11 has a trapezoidal shape, the two bases (the upper base 11d and the lower base 11e) or the two legs 11f are typically regarded as the lateral surfaces 11s located on both sides with the radiation surface 11a therebetween; nevertheless, one of the two bases (the upper base 11d or the lower base 11e) and one of the two legs 11f may be regarded as the lateral surfaces 11s located on both sides with the radiation surface 11a therebetween. In a case where the radiation surface 11a has a triangular shape, any two sides may be regarded as the lateral surfaces located on both sides. In a case where the radiation surface 11a has a circular shape, two arcs constituting the circumference of a circle may be regarded as the lateral surfaces located on both sides.

The first adhesive 37 may be applied to any desired part of the supporter 13. The area of bonding between the first adhesive 37 and the lateral surface 11s of the plate-like element 11 (or the lateral surface 21s in the present embodiment) may be essentially analogous to the area of bonding between the first adhesive 37 and the supporter 13, where the direction in which the periphery of the plate-like element 11 extends (i.e., the circumferential direction) is concerned. As mentioned above, the first adhesive 37 may be applied to the lateral surfaces 11s in a manner so as to extend along the entire periphery of the plate-like element 11 or in a manner so as to extend along only part of the periphery of the plate-like element 11 and may be applied to the lateral surfaces 11s located on both sides with the radiation surface 11a therebetween. The same may go for the area of bonding between the first adhesive 37 and the supporter 13 and/or the overlap between this area and the area of bonding between the first adhesive 37 and the lateral surfaces 11s.

When viewed in cross section, the first adhesive 37 in the illustrated example is located on only part of a surface of the overlapping portion 13e that is closer than the reverse surface to the plate-like element 11; that is, the first adhesive 37 is located on only part of a bottom surface of the recess 13r. More specifically, the first adhesive 37 is applied to a region that is not hidden from view by the plate-like element 11. Some discretion is allowed as to the proportion of the area coated with the first adhesive 37 in the total area of this region. For example, the first adhesive 37 may be applied to the entirety of the region concerned. Alternatively, the first adhesive 37 may be applied to only part of the region that is closer than the rest of the region to the plate-like element 11 (the opening 13h), as in the illustrated example. Still alternatively, air may be put into the first adhesive 37 on the overlapping portion 13e such that the first adhesive 37 is applied to only part of the region that is farther than the rest of the region from the plate-like element 11.

In another example (not illustrated), the first adhesive 37 may be applied to the wall surface and the bottom surface of the recess 13r or to the wall surface of the recess 13r only or may be applied to a surface in which an opening is defined by the recess 13r (e.g., a top surface of the partition portion 13f). In the height direction of the wall surface of the recess 13r, the first adhesive 37 may be applied to the entirety of the wall surface or may be applied to only part of the wall surface. In the latter case, the region extending in the height direction of the wall surface and coated with the first adhesive 37 may be more than or equal to one half of the wall surface or may be less than one half of the wall surface. In the case where the first adhesive 37 is applied to only part of the wall surface, the region being part of the wall surface and coated with the first adhesive 37 may be on or close to the bottom surface of the recess 13r or may be located opposite the bottom surface of the recess 13r.

The first adhesive 37 may be made of an organic material or an inorganic material. Furthermore, the first adhesive 37 may be made of an insulating material or a conductive material. For example, the first adhesive 37 may be made of resin or metal. The elastic modulus of the material of the first adhesive 37 is not limited to a particular value and may be lower than or higher than the elastic modulus of the material of the supporter 13. The term "elastic modulus" may herein refer to the longitudinal elastic modulus (Young's modulus, longitudinal elastic coefficient), and the value of the elastic modulus at the median of the anticipated operating temperature range of the generation part 7 may serve as a reference. The same applies to the following.

The first adhesive 37 may be an adhesive agent (e.g., an elastic adhesive) that is elastic after setting. For example, the first adhesive 37 may be a silicone-based adhesive agent or a urethane-based adhesive agent. Examples of the adhesive agent include one-part adhesives and two-part adhesives. The first adhesive 37 may be an elastic material that yields an elongation of 35% or more when being torn in a tensile test after setting.

### Flexible Substrate

The FPC 35 (flexible substrate), which is illustrated in Fig. 6, is electrically connected to the plate-like element 11 (or, more specifically, to the element substrate 19). The FPC 35 aids in transmission of signals between the plate-like element 11 and the device main body 5.

For example, the FPC 35 includes an insulating film (not illustrated) and a conductor layer (not illustrated) on the film. All in all, the FPC 35 is flexible. The FPC 35 may be a member of a specific desired structure made of any desired material and having desired dimensions. The FPC 35 may optionally have an electronic component (e.g., an integrated circuit (IC)) mounted thereon.

FPC 35 and the cavity member 21 are located on opposite sides with the element substrate 19 therebetween. The FPC 35 may be large enough to extend across all of the vibratory elements 15 included in one plate-like element 11. The FPC 35 may be large enough to extend over only one plate-like element 11 as in the illustrated example or may be large enough to extend over more than one plate-like element 11 or all of the plate-like elements included in the generation part 7. In the former case, the area of the FPC 35 may be greater than, equal to, or less than the area of the plate-like element 11. The FPC 35 viewed in plan may be geometrically similar or analogous to the plate-like element 11 viewed in plan or may be geometrically different from it.

The second conductor layer 29 (see Fig. 4) of the element substrate 19 includes extended electrodes (not illustrated). The electrodes extend from the second electrodes 33 above the cavities 21c to the region that does not overlap the cavities 21c. In the example illustrated in Fig. 6, the region concerned and each cavity 21c do not coincide with each other in a direction passing through the drawing plane. The FPC 35 includes pads (not illustrated) that face the extended electrodes. The extended electrodes are bonded to the pads with bumps 39 therebetween. The bumps 39 are electrically conductive. The second electrodes 33 are electrically connected to the FPC 35 accordingly. Through-conductors (not illustrated) extending through the piezoelectric layer 27 may form a connection between the first electrode 31 of the element substrate 19 and terminals (not illustrated) that are exposed at the surface of the element substrate 19 in a manner so as to face the FPC 35. The terminals may be connected to the pads of the FPC 35 with the bumps 39 therebetween.

The bumps 39 may be made of solder or a conductive adhesive. The solder may be lead-free solder. The conductive adhesive may be resin containing conductive particles. The extended electrodes, the pads, and the bumps 39 each may have any specific desired shape and desired dimensions.

With the given thickness of the bumps 39 (and the given thickness of the pads bonded to the bumps 39), a space 71 is provided between the element substrate 19 and the FPC 35. The space 71 may have any desired thickness. When seen from another perspective, the bumps 39 may have any desired thickness. The thickness of the space 71 is such that the element substrate 19 would be kept from contact with the FPC 35 if the maximum anticipated bending load is exerted on the vibratory elements 15 toward the FPC 35. The thickness of the space 71 may be greater than, equal to, or less than the thickness of the FPC 35. In a case where the thickness of the space 71 is not constant due to the bending of the FPC 35, the minimum thickness, the average thickness, or the maximum thickness of the space 71 may be regarded as the aforementioned height of the space 71.

### Example Dimensions

As mentioned above, ultrasonic waves of any desired frequency may be generated by the radiator 3, and the constituent parts of the radiator 3 each may have desired dimensions. Specific examples are as follows. The radiator 3 may generate ultrasonic waves in the frequency range of 0.5 MHz to 2 MHz. The diameter of the generation part 7 (including the periphery of the concave surface 7a and viewed in plan) may be greater than or equal to 50 mm and less than or equal to 200 mm. The equivalent circle diameter of each of the plate-like elements 11 or one of the four sides of the trapezoidal shape of each of the plate-like elements 11 may be greater than or equal to 5 mm and less than or equal to 20 mm. The equivalent circle diameter of each of the vibratory elements 15 may be greater than or equal to 0.2 mm and less than or equal to 2 mm. The thickness of the element substrate 19 may be greater than or equal to 50 µm and less than or equal to 200 µm. The thickness of the vibration layer 23 and the thickness of the piezoelectric layer 27 each may be greater than or equal to 20 µm and less than or equal to 100 µm, where it is required that there be no contradiction between the thickness of each of these layers and the thickness of the element substrate 19. The thickness of the first conductor layer 25 (the first electrode 31) and the thickness of the second conductor layer 29 (the second electrodes 33) each may be greater than or equal to 0.05 µm and less than or equal to 5 µm. The thickness of the supporter 13 may be greater than or equal to the thickness of the element substrate 19.

### Bag

Referring back to Fig. 1, the bag 9 may have any desired shape and any desired size and may be made of any desired material. For example, the bag 9 is spherical in shape; that is, the bag 9 as a whole bulges outward. In a case where the radiator 3 is designed for a particular part of a human body, the bag 9 may have a projection and/or a recess conforming to the shape of a recess and/or a projection of the particular part.

The bag 9 is made of a material that is at least impermeable to the liquid LQ (impervious to water) and flexible. The material of the bag 9 may be elastic. For example, the material of the bag 9 may be a thermosetting elastomer (known as rubber), a thermoplastic elastomer (elastomer in a narrow sense), or resin that does not contain such an elastomer (resin in a narrow sense) and that is flexible. Examples of the thermosetting elastomer include vulcanized rubber (rubber in a narrow sense) and thermosetting resin elastomers.

As mentioned above, the liquid LQ is sealed in the bag 9, at least while the ultrasonic device 1 in operation. The bag 9 (the radiator 3) may come sealed with the liquid LQ inside, or the bag 9 may be filled with the liquid LQ and sealed at the time of being used. The bag 9 (the radiator 3) may be optionally fitted with a port that can be opened and closed to pour the liquid LQ into the bag 9 (and/or to drain the liquid LQ out of the bag 9). The port may be structurally analogous to various well-known open/close mechanisms.

The bag 9 has an opening 9a, which faces the generation part 7. A portion of the generation part 7 or, more specifically, a portion including the concave surface 7a is in contact with the liquid LQ in the bag 9 through the opening 9a. The other portion of the generation part 7 or, more specifically, a portion including the surface opposite to the concave surface 7a is in contact with the ambient gas (e.g., air) outside the radiator 3, not with the liquid LQ in the bag 9. Thus, the first surfaces 15a of the vibratory elements 15, that is, the surfaces oriented toward the patient 101 are in contact with the liquid LQ, and the reverse surfaces (i.e., the second surfaces 15b of the vibratory elements 15) are in contact with gas (e.g., air). Any desired mechanism may be adopted to reduce the possibility that the liquid LQ will leak out between the peripheral portion of the opening 9a and the generation part 7.

### Device Main Body

The device main body 5 includes mainly a drive control unit 41, a transfer unit 43, an input unit 45, and an output unit 47. The drive control unit 41 sets the radiator 3 in operation and controls the operation of the radiator 3. The transfer unit 43 causes the radiator 3 to move from one place to another. The input unit 45 accepts an input operation performed by the user. The output unit 47 provides information to the user.

For example, a cable 49 forms an electrical connection between the drive control unit 41 and electrical circuitry of the generation part 7. The electrical circuitry includes, for example, the FPC 35. The drive control unit 41 includes a drive unit 51 and a control unit 53. The drive unit 51 inputs, into the generation part 7, a signal for generation of ultrasonic waves. The control unit 53 controls the drive unit 51.

The task of inputting a driving signal into the first electrode 31 and the second electrodes 33 to generate ultrasonic waves may be shared by the drive unit 51 and the electrical circuitry of the generation part 7 as appropriate. For convenience, an embodiment will be described below in which the electrical circuitry of the generation part 7 is solely committed to transmitting a signal from the drive unit 51 to the first electrode 31 and the second electrodes 33. In some embodiments, the function of the drive unit 51, which will be described below, is at least partially assumed by the generation part 7.

The drive unit 51 converts power (e.g., commercial power) into alternating current having a waveform (e.g., frequency and voltage (amplitude)) specified by the control unit 53 and inputs the alternating current power into the first electrode 31 and the second electrodes 33. The driving signal is alternating current power that is substantially equal in frequency to the ultrasonic waves to be emitted and has a voltage corresponding to the amplitude of the ultrasonic waves to be emitted. The driving signal may, for example, be in the form of a rectangular wave (pulse), a sign wave, a triangular wave, or a sawtooth wave.

The control unit 53 includes a computer (not illustrated). The computer includes mainly a central processing unit (CPU), read-only memory (ROM), random-access memory (RAM), and an external storage device. The CPU executes programs stored in the ROM or the external device to implement a functional unit that performs various kinds of control. The control unit 53 sets, on the basis of a signal from the input unit 45, the waveform (e.g., frequency and voltage (amplitude)) of a driving signal that will be output by the drive unit 51. The control unit 53 also controls the drive unit 51, which starts or stops outputting a driving signal accordingly.

The transfer unit 43 includes a holding mechanism (not illustrated) and a driving source (not illustrated). The holding mechanism holds the radiator 3. The driving source, which may be a motor, supplies the holding mechanism with power for causing the radiator 3 to move from one place to another. The transfer unit 43 may be configurationally analogous to an articulated robot, a SCARA robot, or a Cartesian robot. The transfer unit 43 causes the radiator 3 to move relative to the patient 101 on the basis of a control command given by the control unit 53. The relative movement of the radiator 3 may be such that the radiator 3 is brought close to the patient 101 and/or is positioned to focus the ultrasonic waves onto the lesion 103. The control unit 53 controls the transfer unit 43 on the basis of a signal from the input unit 45 and/or a signal from a sensor (not illustrated) that locates the lesion 103. The transfer unit 43 or the driving source of the transfer unit 43 may be omitted, in which case man power may be used to shift the radiator 3 from one place to another and to put the radiator 3 in place.

The input unit 45 includes mainly a keyboard, a mouse, a mechanical switch, and/or a touch panel. The input unit 45 accepts, for example, an operation for setting the frequency and the amplitude of ultrasonic waves to be emitted by the radiator 3 and an operation for instructing the radiator 3 to start or stop emitting ultrasonic waves. The output unit 47 includes mainly a display and/or a speaker. The output unit 47 provides information about the current settings, such as the frequency and the amplitude of ultrasonic waves.

As mentioned above, the ultrasonic radiator 3 in the present embodiment includes the plate-like elements 11, the supporter 13, and the first adhesive 37. The plate-like elements 11 each have, on the front side or the back side, the radiation surface 11a from which ultrasonic waves are emitted. The supporter 13 holds the plate-like elements 11 in such a manner that the respective radiation surfaces 11a in different directional orientations are directed toward the same location (the lesion 103). The plate-like elements 11 are bonded to the supporter 13 with the first adhesive 37. The plate-like elements 11 each include the vibratory elements 15 that are variously located in the radiation surface 11a and that generate ultrasonic waves. The first adhesive 37 is applied to the lateral surfaces 11s of each of the plate-like elements 11.

Thus, the concave surface 7a, from which ultrasonic waves are emitted, is not in itself is an integral whole and is composed of the plate-like elements 11. This provides ease of producing the generation part 7. Each plate-like element 11 may, for example, be in the form of a flat plate, in which case the plate-like element 11 can be prepared in much the same way as commonly used circuit boards.

As mentioned above, the plate-like elements 11 each include the vibratory elements 15. This feature provides greater design flexibility to the plate-like elements 11. This will be described below in more detail. Supposing that each plate-like element 11 was designed to vibrate as a whole, an increase in the area of the plate-like element 11 would translate into a decrease in the resonant frequency of the plate-like element 11. In light of the need to bring the frequency of the plate-like elements 11 in a predetermined vibration mode close to a desired driving frequency of ultrasonic waves, there is a limit to the extent to which the size of the plate-like elements 11 can be increased. This problem is addressed in the present embodiment, in which the resonant frequency of the vibratory elements 15 may be brought close to the driving frequency of ultrasonic waves, and such an increase in the area of each plate-like elements 11 can thus be tolerated.

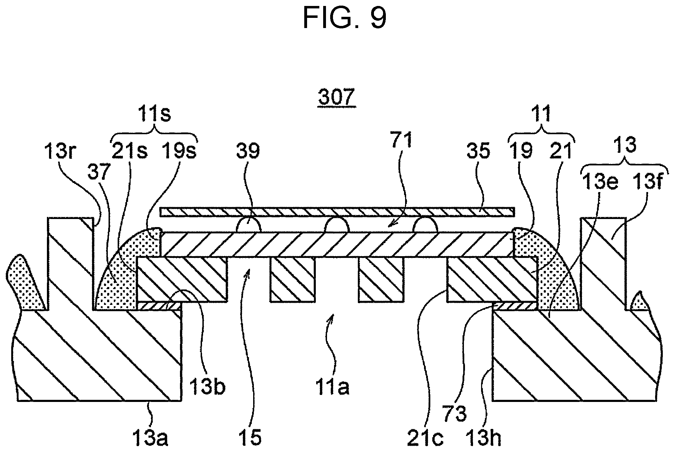

The first adhesive 37 is applied to the lateral surfaces 11s of each of the plate-like elements 11. This provides ease of focusing ultrasonic waves onto the lesion 103 in an efficient manner. This will be described below in more detail. The following describes another embodiment in which the first adhesive 37 is omitted and the supporter 13 and each plate-like element 11 are bonded together only in the overlap between the overlapping portion 13e of the supporter 13 and the plate-like element 11 (see Fig. 9 for a second adhesive 73, which will be described later). In this embodiment, the deformation in the planar direction is restricted in part of each plate-like element 11 or, more specifically, a region closer to the overlapping portion 13e, whereas the deformation in the planar direction is not restricted in a region located opposite the overlapping portion 13e. The plate-like elements 11 each include a layer (e.g., the element substrate 19) that expands and contracts in the planar direction due to vibration for generating ultrasonic waves or due to temperature variations. For this reason, each plate-like element 11 as a whole is prone to bending and deformation. Thus, energy that is supposed to be used to generate ultrasonic waves is dispersed under the influence of bending and deformation caused by vibration for generating ultrasonic waves. If the plate-like elements 11 are bent and deformed for some reason, the vibratory elements 15 of each plate-like element 11 would deviate from the intended orientation direction. As a result, the region onto which ultrasonic waves are focused would be greater than intended. This problem is addressed in the present embodiment, in which the first adhesive 37 is applied to the lateral surfaces 11s of each of the plate-like elements 11 such that the region in which the deformation of each plate-like element 11 in the planar direction is restricted is extended in the thickness direction of the plate-like element 11. Thus, the plate-like elements 11 are less prone to bending and deformation. This leads to low energy loss and reduced expansion of the focal region.

The plate-like elements 11 in the present embodiment each include the element substrate 19 and the cavity member 21. The element substrate 19 extends along the radiation surface 11a and includes the vibratory elements 15. The cavity member 21 extends along the radiation surface 11a and has openings (the cavities 21c). Each of the openings is covered with the corresponding one of the vibratory elements 15. The element substrate 19 includes the piezoelectric layer 27. The piezoelectric layer 27 produces stress along the radiation surface 11a. The first adhesive 37 is applied to the lateral surfaces 21s of the cavity member 21 and/or the lateral surfaces 19s of the element substrate 19.

With the piezoelectric layer 27 being included in the element substrate 19 to produce stress along the radiation surface 11a, the element substrate 19 as a whole is highly likely to expand and contract in the planar direction due to the vibration for generating ultrasonic waves. Thus, it is more likely that there arise the aforementioned problems, such as the dispersion of energy; and where there is more need to address the problems, the aforementioned effects prove more advantageous.

In the present embodiment, the supporter 13, the cavity member 21, and the element substrate 19 are stacked in this order in the direction the radiation surface 11a faces. The first adhesive 37 is applied to the lateral surfaces 21s of the cavity member 21 that are located on both sides with the radiation surface 11a therebetween.

In this case, the cavity member 21 may be bonded to the supporter 13. The cavity member 21 is closer than any other member of the plate-like element 11 to the supporter 13 and is therefore easily bonded. The cavity member 21 is subject to the stress generated by the element substrate 19 in the planar direction and exerted on only one of two surfaces of the cavity member 21 that are opposite in the thickness direction. For this reason, the cavity member 21 is prone to bending and deformation. Applying the first adhesive 37 to the lateral surfaces 21s of the cavity member 21 effectively produces the aforementioned effect of reducing the degree of bending and deformation. The displacement of the lateral surfaces 21s that are located on both sides with the radiation surface 11a therebetween, in particular, is restricted by the first adhesive 37 such that the degree of deformation (expansion and contraction) of the cavity member 21 in the planar direction is effectively reduced.

The plate-like elements 11 in the present embodiment are each in the form of a flat plate.

As mentioned above, the plate-like elements 11 each being in the form of a flat plate can be produced in much the same way as commonly used circuit boards. This provides ease of producing the generation part 7. This geometrical feature also offers an advantage that ultrasonic waves are focused onto a relatively wide area.

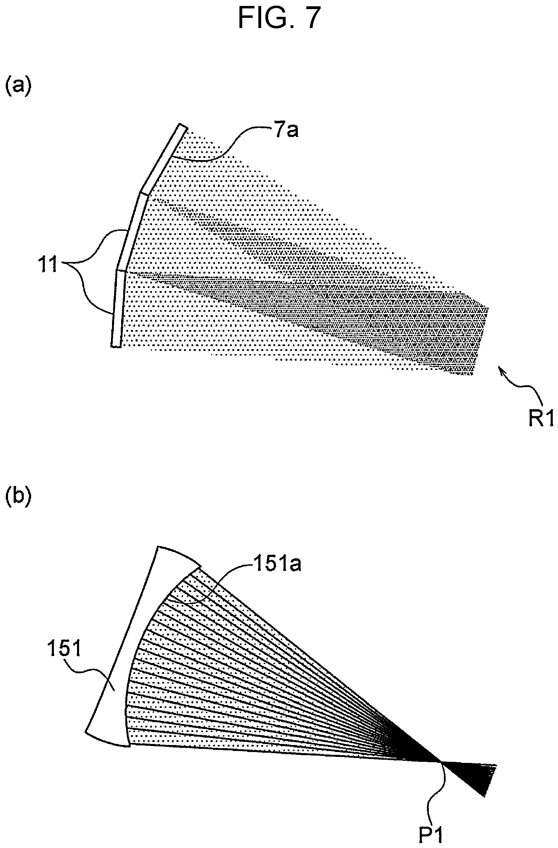

Figs. 7(a) and 7(b) are schematic diagrams for explanation of the effects of focusing ultrasonic waves onto a relatively wide area. More specifically, Fig. 7(a) schematically illustrates how ultrasonic waves in the present embodiment are focused, and Fig. 7(b) schematically illustrates how ultrasonic waves in another example are focused.

Referring to Fig. 7(b), an element 151 in the example concerned has a radiation surface 151a, which is curved. The radiation surface 151a is a lens member that is in the form of a monolithic plate and provided with the vibratory elements 15. To be more precise, elements (not illustrated) analogous to the vibratory elements 15 are arranged behind the lens member. The lens member brings ultrasonic waves generated by the vibratory elements 15 to a focal point P1. The focal point P1 is in theory a point or a relatively small area where ultrasonic waves meet.

Referring to Fig. 7(a), ultrasonic waves emitted from the plate-like elements 11 are brought to a focal region R1, ideally with no change in (beam) width. Ultrasonic waves from the plate-like elements 11 are then brought into focus. In theory, the focal region R1 is thus substantially equal in width to each of the ultrasonic waves emitted from the plate-like elements 11. The intensity of ultrasonic waves is substantially constant within the width of the focal region R1. These features of the focal region R1 bring efficiency and/or safety to the treatment of the lesion 103 of certain size and the treatment of diseases of particular types.

As mentioned above, the plate-like elements 11 in the present embodiment each include the vibratory elements 15. This feature is advantageous in that the area of each plate-like element 11 can be set independently of the frequency of ultrasonic waves (independently of the resonant frequency of the vibratory elements 15). It is thus easy to ensure that the desired ultrasonic frequency and the desired beam width (the plate-like elements 11 having the desired area) are mutually compatible. Each plate-like element 11 as a whole is less prone to bending and deformation, as mentioned above. It is thus easily ensured that the plate-like elements 11 emit ultrasonic waves of constant width.

### Second Embodiment

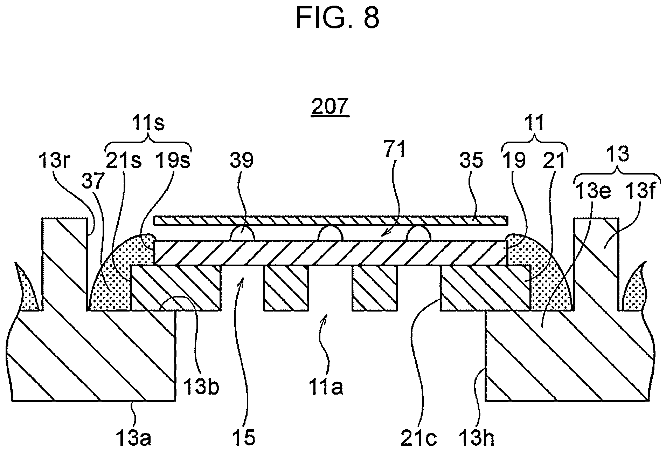

Fig. 8 is a sectional view analogous to Fig. 6 and illustrates a generation part 207 according to a second embodiment.

The present embodiment differs from the first embodiment in that the first adhesive 37 is applied not only to the lateral surfaces 21s of the cavity member 21 but also to the lateral surfaces 19s of the element substrate 19. The first adhesive 37 may be applied not only to the lateral surfaces 19s but also to another surface of the element substrate 19 (e.g., a surface located opposite the supporter 13) as in the illustrated example. Applying the first adhesive 37 to such a surface is not a must. The area of bonding between the first adhesive 37 and the supporter 13 along the periphery of the plate-like element 11 and the area of bonding between them viewed in cross section may be understood as analogous to the relevant description of the first embodiment.

In the thickness direction of the plate-like element 11, the first adhesive 37 may be applied to the entirety of the lateral surface 19s of the element substrate 19 as in the illustrated example or may be applied to only part of the lateral surface 19s. In the latter case, some discretion is allowed as to the proportion of the region coated with the first adhesive 37 in the total area of the lateral surface 19s. For example, the region extending in the thickness direction of the plate-like element 11 and coated with the first adhesive 37 may be more than or equal to one half of the lateral surface 19s or may be less than one half of the lateral surface 19s. The first adhesive 37 may be applied to any desired part of the lateral surface 19s; that is, the region being part of the lateral surface 19s and coated with the first adhesive 37 may be on or close to the cavity member 21 or may be located opposite the cavity member 21.

The first adhesive 37 in the illustrated example is applied to the lateral surface 21s of the cavity member 21 and to the lateral surface 19s of the element substrate 19 in a manner so as to cover the entirety of each of these lateral surfaces. Alternatively, the first adhesive 37 may be applied to (all or part of) the lateral surface 19s only or may be applied to (all or part of) the lateral surface 19s and to part of the lateral surface 21s. For example, air may be put into the first adhesive 37 on the overlapping portion 13e such that the first adhesive 37 is applied to the lateral surface 19s only or to part of the lateral surface 21s and to (all or part of) the lateral surface 19s.

The area of bonding between the first adhesive 37 and the lateral surface 19s may be understood as analogous to the area of bonding between the first adhesive 37 and the lateral surface 11s (or, more specifically, the lateral surface 21s) in the first embodiment, where the direction in which the periphery of the plate-like element 11 extends is concerned. For example, the first adhesive 37 may be applied to the lateral surfaces 19s in a manner so as to extend along the entire periphery of the plate-like element 11 or in a manner so as to extend along only part of the periphery of the plate-like element 11. The first adhesive 37 may be applied to the lateral surfaces 19s located on both sides with the radiation surface 11a (or, more specifically, at least one vibratory element 15) therebetween.

As mentioned above, the first adhesive 37 in the present embodiment is applied to the lateral surfaces 11s of each of the plate-like elements 11. This feature produces effects equivalent to those produced in the first embodiment. More specifically, the present embodiment produces the effect of reducing the degree of bending and deformation of the entirety of each plate-like element 11, the effect of reducing energy loss, and the effect of reducing the possibility that the focal region R1 will be greater than intended.

The first adhesive 37 in the present embodiment is applied not only to the lateral surfaces 21s of the cavity member 21 but also to the lateral surfaces 19s of the element substrate 19 that are located on both sides with the radiation surface 11a therebetween.

This feature enhances the aforementioned effects. One of the reasons for this may be that the area of bonding is greater in the present embodiment than in the first embodiment. Another reason may be that the element substrate 19 (the piezoelectric layer 27) as a whole is less prone to expansion and contraction, which would otherwise cause bending and deformation of the entirety of each plate-like element 11.

### Third Embodiment

Fig. 9 is a sectional view analogous to Fig. 6 and illustrates a generation part 307 according to a third embodiment.

The present embodiment differs from the first and second embodiments in that each plate-like element 11 is coated not only with the first adhesive 37 but also with a second adhesive 73, with which each plate-like element 11 and the supporter 13 are bonded together in the direction the radiation surface 11a faces. Referring to Fig. 9, the region coated with the first adhesive 37 is much the same in the present embodiment and the second embodiment. Alternatively, the region coated with the first adhesive 37 may be much the same in the present embodiment and the first embodiment.

As mentioned above, the plate-like element 11 is oriented in such a manner that the cavity member 21 faces the overlapping portion 13e of the supporter 13. More specifically, the peripheral portion of the cavity member 21 and the overlapping portion 13e of the supporter 13 overlap each other in the direction the radiation surface 11a faces. The second adhesive 73 is located between the peripheral portion of the cavity member 21 and the overlapping portion 13e of the supporter 13, which are bonded together accordingly.

The second adhesive 73 may be applied to any desired part of the region concerned. For example, the second adhesive 73 may be applied to the entirety of the overlap between the plate-like element 11 (or, more specifically, the cavity member 21) and the overlapping portion 13e of the supporter 13, with the overlap extending in the direction away from the midsection of the plate-like element 11 toward the periphery of the plate-like element 11. Alternatively, the second adhesive 73 may be applied to only part of the overlap. In the latter case, the region coated with the second adhesive 73 may include the inner edge of the overlap (the opening 13h) or the outer edge of the overlap or may be discretely located away from both the inner edge and the outer edge. The second adhesive 73 may lie off the outer edge of the plate-like element 11. In this case, the second adhesive 73 in at least part of the overlap between the first adhesive 37 and the overlapping portion 13e may be located between them.

The area of bonding between the second adhesive 73 and the plate-like element 11 and the area of bonding between the second adhesive 73 and the overlapping portion 13e of the supporter 13 may be understood as analogous respectively to the area of bonding between the first adhesive 37 and the plate-like element 11 in the first embodiment and the area of bonding between the first adhesive 37 and the supporter 13 in the first embodiment, where the direction in which the periphery of the plate-like element 11 extends is concerned. For example, the second adhesive 73 may be applied in a manner so as to extend along the entire periphery of the plate-like element 11 or in a manner so as to extend along only part of the periphery of the plate-like element 11. The first adhesive 37 may be applied to both the plate-like element 11 and the overlapping portion 13e in a manner so as to be located on both sides with the radiation surface 11a (or, more specifically, at least one vibratory element 15) therebetween.

The second adhesive 73 may be applied in any desired thickness. For example, the thickness of the second adhesive 73 may be greater than, equal to, or less than one half of the thickness of the plate-like element 11.

The second adhesive 73 and the first adhesive 37 may be made of the same material or may be made of different materials. In either case, the material of the second adhesive 73 may be understood as analogous to the aforementioned material of the first adhesive 37.

In the case where the first adhesive 37 and the second adhesive 73 are made of different materials, the elastic modulus of the material of the first adhesive 37 may be higher than the elastic modulus of the material of the second adhesive 73. Alternatively, the elastic modulus of the material of the first adhesive 37 may be lower than the elastic modulus of the material of the second adhesive 73. To that end, various materials may be used in combination. For example, the material of higher elastic modulus may be a commonly used resin adhesive, and the material of lower elastic modulus may be the elastic adhesive mentioned above. Materials with any desired degree of difference in elasticity may be used. For example, the elastic modulus of one material is not lower than two times the elastic modulus of the other material, not lower than five times the elastic modulus of the other material, or not lower than ten times the elastic modulus of the other material.

As mentioned above, the first adhesive 37 in the present embodiment is applied to the lateral surfaces 11s of each of the plate-like elements 11. This feature produces effects equivalent to those produced in the first embodiment.

In the present embodiment, the supporter 13 overlaps the peripheral portions of the plate-like elements 11 in the direction the radiation surfaces 11a of the plate-like elements 11 face, with the second adhesive 73 being located between the supporter 13 and each of the peripheral portions.

This layout leads to an increase in the area of bonding between each plate-like element 11 and the supporter 13. The increased area of bonding reduces the possibility that the stress caused by vibration will concentrate in the first adhesive 37, and the durability to withstand vibration will be increased accordingly.

In the present embodiment, the elastic modulus of the first adhesive 37 may be higher than the elastic modulus of the second adhesive 73.

In this case, the first adhesive 37 is harder than the second adhesive 73. It is thus easily ensured that the effects described above in relation to the first embodiment will not wear off soon. Conversely, the second adhesive 73 is softer than the first adhesive 37 such that displacement between the surface of the plate-like element 11 and the surface of the supporter 13 in the overlap between the plate-like element 11 and the overlapping portion 13e may be tolerated to a certain extent. The second adhesive 73 will be less likely to come off the plate-like element 11 or the overlapping portion 13e. This will result in improved airtightness and bonding strength.

In the present embodiment, the converse of the above is possible where the elastic modulus of the first adhesive 37 may be lower than the elastic modulus of the second adhesive 73.

In this case, the regions conducive to acting as fixed ends are closer than the lateral surfaces 11s coated with the first adhesive 37 to the midsection of the plate-like element 11. This means that the distance between the fixed ends is short, and as a result, the plate-like element 11 operable in various vibration modes produces resonance in the upper range of the frequency spectrum. For example, the resonant frequency in any one of the vibration modes in which the plate-like element 11 is designed to operate may be close to the frequency of ultrasonic waves and may be in the upper range of the frequency spectrum. In this case, using a harder material as the second adhesive 73 is advantageous in that the resonant frequency is shifted away from the frequency of ultrasonic waves into a higher frequency range. Consequently, ultrasonic waves are rendered less susceptible to unwanted vibrations.

### Fourth Embodiment

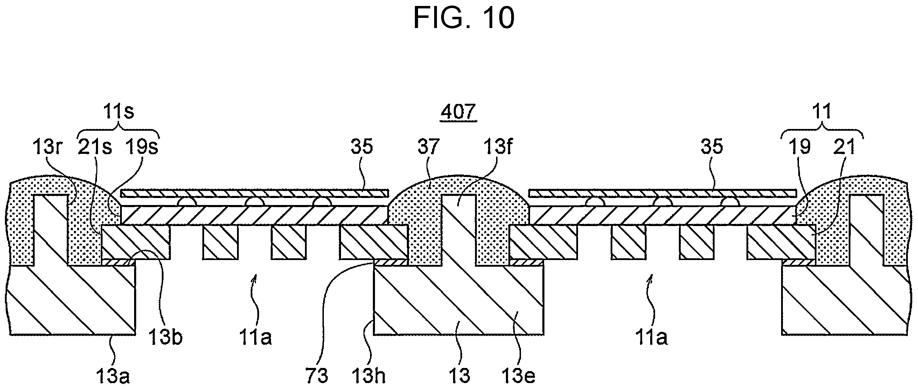

Fig. 10 is a sectional view analogous to Fig. 6 and illustrates a generation part 407 according to a fourth embodiment. Two plate-like elements 11 that are adjacent to each other lie in the range illustrated in Fig. 10, in which the angular difference between the two plate-like elements 11 is left out of consideration.

The direction in which the plate-like elements 11 are arranged side by side (in the left-and-right direction in Fig. 10) may be parallel to the concave surface 7a (or, more locally, the radiation surface 11a). More specifically, the direction concerned may coincide with the radial direction of the concave surface 7a or the circumferential direction of the concave surface 7a. When the plate-like elements 11 each have a specific planar shape or are in a specific arrangement, the direction concerned does not necessarily coincide with any of these directions. The layout illustrated in Fig. 10 applies to only one direction or to every direction (e.g., both the radial direction and the circumferential direction). The layout illustrated in Fig. 10 also applies to either all or only some of the plate-like elements 11 arranged side by side in the direction that coincides with the left-and-right direction in Fig. 10.

In the present embodiment, two adjacent lateral surfaces 11s each being is a lateral surface of the corresponding one of two adjacent elements of the plate-like elements 11 are bonded together with the first adhesive 37. Although the first adhesive 37 with which the two adjacent lateral surfaces 11s are bonded together is regarded as one adhesive, it may be construed that the two lateral surfaces 11s are coated with the respective first adhesives 37 sticking to each other.

The supporter 13 in the present embodiment includes the partition portion 13f located between two adjacent lateral surfaces 11s, and the wall surface and the top surface of the partition portion 13f are coated with the first adhesive 37. In some embodiments (not illustrated), the supporter 13 does not include the partition portion 13f, in which case the first adhesive 37 is applied to the lateral surfaces 11s, not to the partition portion 13f, in a manner so as to be located between two adjacent lateral surfaces 11s.

The first adhesive 37 in the illustrated example is applied to the entirety of the wall surface extending in the height direction of the partition portion 13f and is also applied to the two adjacent lateral surfaces 11s. As can be understood from the above description of the first embodiment, the first adhesive 37 may be applied to only part of the wall surface (e.g., a top part of the wall surface) and to the two adjacent lateral surfaces 11s. The first adhesive 37 may be applied in any desired thickness, where the thickness of the first adhesive 37 between the two adjacent lateral surfaces 11s is concerned. In the present embodiment, the thickness is inclusive of the height of the partition portion 13f. For example, the thickness of the first adhesive 37 may be greater than or equal to the thickness of the plate-like element 11 as in the illustrated example or may be less than the thickness of the plate-like element 11.

The height of the partition portion 13f may be such that an upper surface of the partition portion 13f is located at a level above an upper surface of the element substrate 19 included in the plate-like element 11 (i.e., at a level above a surface oriented toward the FPC 35) and at a level below a lower surface of the FPC 35 (i.e., at a level below a surface oriented toward the element substrate 19). The partition portion 13f of this height reduces interference of vibrations of the element substrates 19 of the two adjacent plate-like elements 11. Furthermore, the partition portion 13f of this height is less likely to cause bending of the FPC 35 located at a level above the partition portion 13f and is thus less likely to place strain on the FPC 35. This is preferable in terms of the handleability of the FPC 35 in the process of bonding the FPC 35 to the element substrate 19.

The illustrated example involves the use of the second adhesive 73, which has been described above in relation to the third embodiment. The first adhesive 37 is applied to the lateral surfaces 21s of the cavity member 21 and to the lateral surfaces 19s of the element substrate 19, as in the second embodiment. The second adhesive 73 may be eliminated, or the first adhesive 37 may be applied to the lateral surfaces 19s only or to the lateral surfaces 21s only. In any case, two adjacent lateral surfaces 11s may be bonded together with the first adhesive 37, irrespective of the presence of the partition portion 13f.

As mentioned above, the first adhesive 37 in the present embodiment is applied to the lateral surfaces 11s of each of the plate-like elements 11. This feature produces effects equivalent to those produced in the first embodiment. More specifically, the present embodiment produces the effect of reducing the degree of bending and deformation of the entirety of each plate-like element 11, the effect of reducing energy loss, and the effect of reducing the possibility that the focal region R1 will be greater than intended.

In the present embodiment, two adjacent lateral surfaces 11s each being a lateral surface of the corresponding one of two adjacent elements of the plate-like elements 11 are bonded together with the first adhesive 37.

This feature enhances the aforementioned effect of reducing bending and distortion. This will be described below in more detail. Two adjacent plate-like elements 11 usually contract or stretch in tandem with each other. In a state in which the two adjacent lateral surfaces 11s are bonded together, the plate-like elements 11 contract in such a manner that the two adjacent lateral surfaces 11s pull each other with the first adhesive 37 therebetween, and the plate-like elements 11 stretch in such a manner that the two adjacent lateral surfaces 11s push each other with the first adhesive 37 therebetween. The amount of displacement of the lateral surfaces 11s is thus reduced, and the aforementioned effects are enhanced correspondingly.

The supporter 13 in the present embodiment includes the partition portion 13f. The partition portion 13f is located between two adjacent lateral surfaces 11s each being a lateral surface of the corresponding one of two adjacent elements of the plate-like elements 11. The partition portion 13f is at least partially coated with the first adhesive 37. The elastic modulus of the supporter 13 may be higher than the elastic modulus of the first adhesive 37.

In this case, the two adjacent lateral surfaces 11s pull each other or push each other with the partition portion 13f therebetween, thus coming close to each other or moving away from each other with a further reduced degree of freedom of motion owing to the fact that the elastic modulus of the partition portion 13f is higher than the elastic modulus of the first adhesive 37. The aforementioned effects are enhanced correspondingly.

### Fifth Embodiment

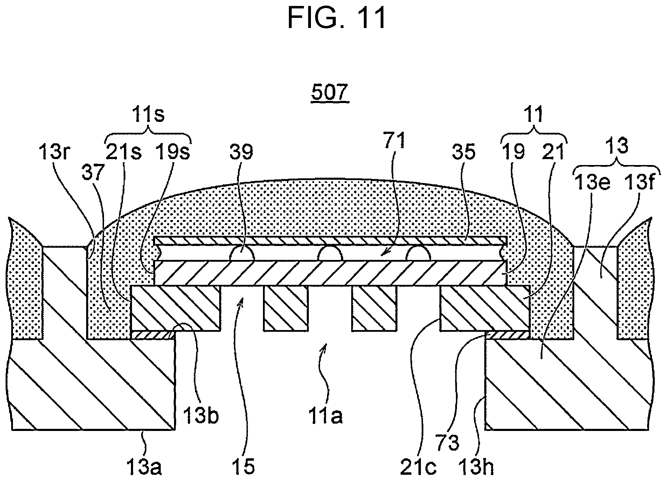

Fig. 11 is a sectional view analogous to Fig. 6 and illustrates a generation part 507 according to a fifth embodiment.

The present embodiment differs from the embodiments described above in that the region coated with the first adhesive 37 extends to the FPC 35. More specifically, the first adhesive 37 on the lateral surfaces 11s of the plate-like element 11 extends off edges of the lateral surfaces 11s to the FPC 35 and is applied to the FPC 35 to close at least part of a peripheral portion of the space 71 between the plate-like element 11 and the FPC 35. For example, the first adhesive 37 is applied all along the periphery of the space 71 to close the peripheral portion of the space 71 such that the space 71 is hermetically sealed.

The peripheral portion of the space 71 may be closed with the first adhesive 37 in any of the following manners: (i) the first adhesive 37 extends off to a surface of the FPC 35 or, more specifically, a surface oriented toward the plate-like element 11; (ii) lateral surfaces of FPC 35 are covered with the first adhesive 37; and (iii) the reverse surface of the FPC 35, that is, a surface located opposite the plate-like element 11 is covered with the first adhesive 37, as in the illustrated example. The reverse surface of the FPC 35, that is, the surface located opposite the plate-like element 11 may be entirely covered with the first adhesive 37 as in the illustrated example, or only part of the surface (e.g., the peripheral portion of the surface) may be covered with the first adhesive 37.

In the case where the FPC 35 is covered with the first adhesive 37, the FPC 35 may be connected to the cable 49 in any desired manner. For example, the FPC 35 may partially extend out of the first adhesive 37 and may be connected directly or indirectly to the cable 49. The state in which the FPC 35 partially extends out of the first adhesive 37 may be herein construed as an example of the state in which the FPC 35 is entirely covered with the first adhesive 37. Wiring connected to the FPC 35 and extending out of the first adhesive 37 may be connected directly or indirectly to the cable 49. In some embodiments, part of one of the surfaces of the FPC 35 or, more specifically, part of the surface located opposite the plate-like element 11 (e.g., the midsection of the FPC 35) is not covered with the first adhesive 37 and is provided with a pad that is connected directly or indirectly to the cable 49.

In an example different from the one illustrated in Fig. 10, the FPC 35 extends over two or more plate-like elements 11, as mentioned above in relation to the first embodiment. In this case as well, the space 71 may be hermetically sealed in such a manner that the first adhesive 37 is applied along the peripheries of the plate-like elements 11 to the surfaces of the FPC 35 or, more specifically, the surfaces oriented toward the plate-like elements 11 and/or is applied along the periphery (e.g., the lateral surfaces) of the FPC 35 extending over two or more plate-like elements 11.

When seen from another perspective, the first adhesive 37 in the present embodiment is located opposite the plate-like element 11 with the FPC 35 therebetween in such a manner that at least the vibratory elements 15 are located within the overlap between the first adhesive 37 and the FPC 35. In the illustrated example, the first adhesive 37 covers the entirety of the FPC 35. Furthermore, the plate-like element 11 is entirely covered with the first adhesive 37 from above the FPC 35. In this respect, the space 71 is or is not hermetically sealed.

The first adhesive 37 on the FPC 35 may be applied in any desired thickness. For example, the thickness of the first adhesive 37 on the FPC 35 may be greater than or equal to the thickness of the plate-like element 11 or may be less than the thickness of the plate-like element 11. An upper surface of the first adhesive 37 or, more specifically, a surface located opposite the FPC 35 may be curved as in the illustrated example or may be flat.

The height of the partition portion 13f in the illustrated example may be such that the upper surface of the partition portion 13f is located at a level above the upper surface of the element substrate 19 included in the plate-like element 11 (i.e., at a level above the surface oriented toward the FPC 35) and at a level below the lower surface of the FPC 35 (i.e., at a level below the surface oriented toward the element substrate 19). The partition portion 13f of this height reduces interference of vibrations of the element substrates 19 of the two adjacent plate-like elements 11. Furthermore, the partition portion 13f of this height is less likely to cause bending of the FPC 35 located at a level above the partition portion 13f and is thus less likely to place strain on the FPC 35. This is preferable in terms of the handleability of the FPC 35 in the process of bonding the FPC 35 to the element substrate 19.

The illustrated example involves the use of the second adhesive 73, which has been described above in relation to the third embodiment. The first adhesive 37 is applied to the lateral surfaces 21s of the cavity member 21 and to the lateral surfaces 19s of the element substrate 19, as in the second embodiment. As in the first to third embodiments, two adjacent lateral surfaces 11s each being a lateral surface of the corresponding one of two adjacent elements of the plate-like elements 11 are not bonded together with the first adhesive 37. In another example, the second adhesive 73 may be eliminated. In still another example, the first adhesive 37 may be applied to the lateral surfaces 19s only or to the lateral surfaces 21s only. In yet still another example, the two lateral surfaces 11s may be bonded together with the first adhesive 37. Two or more of these three examples may be adopted in combination. In any case, the first adhesive 37 may be applied to the FPC 35.

As mentioned above, the first adhesive 37 in the present embodiment is applied to the lateral surfaces 11s of each of the plate-like elements 11. This feature produces effects equivalent to those produced in the first embodiment. More specifically, the present embodiment produces the effect of reducing the degree of bending and deformation of the entirety of each plate-like element 11, the effect of reducing energy loss, and the effect of reducing the possibility that the focal region R1 will be greater than intended.

In the present embodiment, the first adhesive 37 on the lateral surfaces 11s of the plate-like element 11 extends off edges of the lateral surfaces 11s to the FPC 35 and is applied to the FPC 35 to close at least part of the peripheral portion of the space 71 between the plate-like element 11 and the FPC 35.

For example, flow of gas (e.g., air) into or out of the space 71 is restricted due to the fact that at least part of the peripheral portion of the space 71 is closed. One part of each vibratory element 15 or, more specifically, a part including the radiation surface 11a is in contact with the LQ. The other part of each vibratory element 15 or, more specifically, a part including the reverse surface (the surface opposite to the radiation surface 11a) is in contact with the gas in the space 71. That is, the front surface and the back surface of each vibratory element 15 are in contact with the respective substances. For this reason, the symmetry of bending and vibration for generating ultrasonic waves is lost in the vibratory elements 15. As a result, the amount of displacement of the vibratory elements 15 is reduced, and the ultrasound pressure decreases accordingly. In the case where the flow of gas into and out of the space 71 is restricted, the vibratory elements 15 encounter increased resistance from the gas in the space 71. While both surfaces of each vibratory elements 15 encounter the resistance from the respective substances, the difference between the resistance from the liquid LQ and the resistance from the gas in the space 71 is reduced such that the degree of asymmetry of bending and vibration is reduced correspondingly.

In the present embodiment, the plate-like element 11 is entirely covered with the first adhesive 37 from above the FPC 35, and the space 71 is hermetically sealed with the first adhesive 37.

This feature enhances the aforementioned effect of reducing the degree of asymmetry. One of the reasons for this may be that the space 71 is hermetically sealed. Another reason may be that the first adhesive 37 on the FPC 35 makes the FPC 35 less flexible.

### Sixth Embodiment

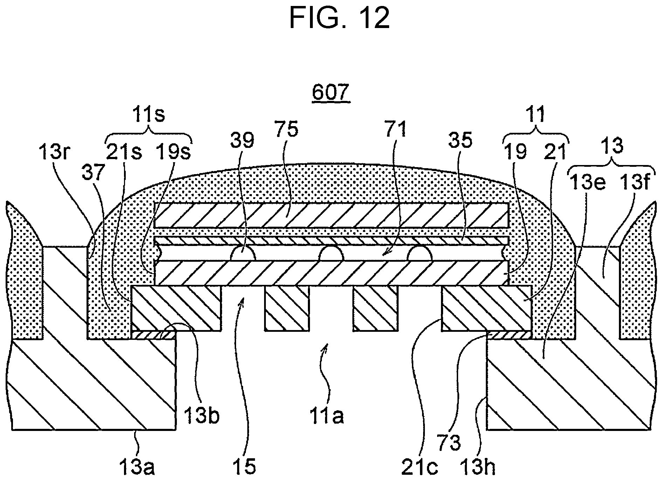

Fig. 12 is a sectional view analogous to Fig. 6 and illustrates a generation part 607 according to a sixth embodiment.

The present embodiment differs from the fifth embodiment in that the FPC 35 faces a reinforcing member 75, which is located opposite the plate-like element 11 with the FPC 35 being disposed between the reinforcing member 75 and the plate-like element 11. Unlike the FPC 35, the reinforcing member 75 is made of an inflexible material. Thus, the FPC 35 is less prone to bending and deformation such that the vibratory elements 15 encounter increased resistance from the gas in the space 71. The aforementioned effect of reducing the degree of asymmetry is achieved accordingly.

The reinforcing member 75 may have any desired shape and desired dimensions. For example, the reinforcing member 75 may be in the form of a flat plate with a constant thickness. The reinforcing member 75 may be large enough to extend across all of the vibratory elements 15 included in one element substrate 19. The area of the reinforcing member 75 may be less than, equal to, or greater than the area of the FPC 35. The reinforcing member 75 viewed in plan may be geometrically similar or analogous to the FPC 35 or the plate-like element 11 viewed in plan or may be geometrically different from it. The thickness of the reinforcing member 75 is greater than the thickness of the FPC 35 as in the illustrated example or may be less than or equal to the thickness of the FPC 35.

The reinforcing member 75 may be made of any desired material. The elastic modulus of the material of the reinforcing member 75 may be higher than the elastic modulus of the material of the first adhesive 37. More specifically, the reinforcing member 75 may be made of metal (e.g., stainless steel), whereas the first adhesive 37 may be a resin adhesive. The reinforcing member 75 and the supporter 13 may be made of the same material or may be made of different materials.

The reinforcing member 75 may extend over the FPC 35 with the first adhesive 37 therebetween as in the illustrated example or may be disposed directly on the FPC 35. In the illustrated example, a surface of the reinforcing member 75 or, more specifically, a surface located opposite the FPC 35 is entirely covered with the first adhesive 37. When seen from another perspective, the reinforcing member 75 is embedded in the first adhesive 37, which covers a front surface, a back surface, and lateral surfaces of the reinforcing member 75. Alternatively, only the peripheral portion of the reinforcing member 75 may be coated with the first adhesive 37, and a surface of the reinforcing member 75 or, more specifically, a surface located opposite the FPC 35 may be entirely or mostly exposed.

As mentioned above, the first adhesive 37 in the present embodiment is applied to the lateral surfaces 11s of each of the plate-like elements 11. This feature produces effects equivalent to those produced in the first embodiment. More specifically, the present embodiment produces the effect of reducing the degree of bending and deformation of the entirety of each plate-like element 11, the effect of reducing energy loss, and the effect of reducing the possibility that the focal region R1 will be greater than intended.

The generation part 607 in the present embodiment includes the reinforcing member 75. The reinforcing member 75 is located opposite the plate-like element 11 with the FPC 35 therebetween. The reinforcing member 75 extends over the FPC 35 with the first adhesive 37 therebetween or is disposed directly on the FPC 35. The reinforcing member 75 is at least partially coated with the first adhesive 37. The elastic modulus of the reinforcing member 75 is higher than the elastic modulus of the first adhesive 37.

For example, making the FPC 35 less prone to bending and deformation is easier in the present embodiment than in an embodiment in which the FPC 35 is covered with the first adhesive 37 only. This feature enhances the aforementioned effect of the fifth embodiment. More specifically, the FPC 35 is less prone to bending and deformation such that the vibratory elements 15 encounter increased resistance from the gas in the space 71. The degree of asymmetry of bending and vibration of the vibratory elements 15 is reduced accordingly.

The technique disclosed herein is not limited to the embodiments described above, and the modifications thereof may be implemented in various forms.

It is not required the plate-like elements be arranged in a manner so as to constitute a concave surface. For example, the plate-like elements may be arranged in like manner with blind slats; that is, the plate-like elements may be arranged in the same plane and form different angles with the plane in a manner so as to be directed toward the same location. It is not required that the plate-like elements 11 each be in the form of a flat plate. The plate-like elements 11 may be curved. In this case, the ultrasonic generator may be designed to focus ultrasonic waves onto a focal point, as illustrated in Fig. 7(b).

It is not required that the vibratory elements (the plate-like elements) each be a piezoelectric element that itself undergoes bending and deformation. For example, the vibratory elements each may include a vibration plate and a piezoelectric element on the back of the vibration plate. The vibration plate serves as a radiation surface. The piezoelectric element expands and contracts in a direction that forms an angle with the radiation surface to cause bending and deformation of the vibration plate. In the case where the vibratory elements are each a piezoelectric element that itself undergoes bending and deformation, the vibratory elements may be unimorphs as in the embodiments above. Alternatively, the vibratory elements may be bimorphs each composed of piezoelectric elements polarized in different direction and stacked in layers. It is not required that the plate-like element each include a cavity member.

In the embodiments above, the supporter has the openings 13h. Through each of the openings 13h, the vibratory elements of the corresponding one of the plate-like elements are exposed. Alternatively, the supporter may have openings each of which is covered with the corresponding one of the vibratory elements. The second adhesive 73 may be applied along the peripheries of the openings to bond the plate-like element to the supporter. In this case as well, applying the first adhesive to the lateral surfaces of the plate-like element provides the effect of helping reduce unwanted vibration, such as vibration propagating through a surface of the plate-like element or, more specifically, through a surface opposite to a surface located on the supporter. The supporter having the openings for the respective vibratory elements eliminates the need for the cavity member; that is, the openings of the supporter dictate the frequency of the vibratory elements just as is the case with the cavity member.

In the embodiments above, the plate-like elements 11 are disposed on the back surface 13b of the supporter 13 (i.e., on the surface located opposite the patient 101). Alternatively, the plate-like elements 11 may be disposed on the front surface 13a of the supporter 13 (i.e., on the surface oriented toward the patient 101). Still alternatively, there may be no overlap between each plate-like element 11 and either of these two surfaces of the supporter 13. Each plate-like element 11 may be oriented in such a manner that either the cavity member 21 or the element substrate 19 of the plate-like element 11 faces the supporter 13 and/or the patient 101.

### Reference Signs List

- 1: ultrasonic device
- 3: ultrasonic radiator
- 11: plate-like element
- 11a: radiation surface
- 11s: lateral surface (of the plate-like element)
- 13: supporter
- 15: vibratory element
- 37: first adhesive
- R1: focal region

## Claims

1. An ultrasonic radiator (3), comprising:
a plurality of plate-like elements (11) each having, on a front side or a back side, a radiation surface (11a) from which ultrasonic waves are emitted;
a supporter (13) that holds the plurality of plate-like elements (11) in an arrangement in which the respective radiation surfaces (11a) in different directional orientations are directed toward the same location; and
a first adhesive (37) with which the plurality of plate-like elements (11) are bonded to the supporter (13), wherein
the plurality of plate-like elements (11) each include a plurality of vibratory elements (15) that are variously located in the radiation surface (11a) and that generate ultrasonic waves,
the plurality of plate-like elements (11) each include
an element substrate (19) that extends along the radiation surface (11a) and includes the plurality of vibratory elements (15), and
a cavity member (21) that extends along the radiation surface (11a) and has a plurality of openings (21c) each of which is covered with a corresponding one of the plurality of vibratory elements (15),
the element substrate (19) includes a piezoelectric layer (27) that produces stress along the radiation surface (11a), and vibrates by regions corresponding to the plurality vibratory elements (15) being bent and distorted,
the supporter (13), the cavity member (21), and the element substrate (19) are stacked in this order in a direction the radiation surface (11a) faces, and
the first adhesive (37) is applied to lateral surfaces (11s) of each of the plurality of plate-like elements (11) and lateral surfaces (21s) of the cavity member (21) that are located on both sides with the radiation surface (11a) therebetween.

2. The ultrasonic radiator (3) according to Claim 1, wherein the first adhesive (37) is applied to the lateral surfaces (21s) of the cavity member (21) and to lateral surfaces (19s) of the element substrate (19) that are located on both sides with the radiation surface (11a) therebetween.

3. The ultrasonic radiator (3) according to claim 1 or 2, wherein the supporter (13) overlaps peripheral portions of the plurality of plate-like elements (11) in a direction that the radiation surfaces (11a) of the plurality of plate-like elements (11) face, with a second adhesive (73) being located between the supporter (13) and each of the peripheral portions.

4. The ultrasonic radiator (3) according to Claim 3, wherein an elastic modulus of the first adhesive (37) is higher than an elastic modulus of the second adhesive (73).

5. The ultrasonic radiator (3) according to Claim 3, wherein an elastic modulus of the first adhesive (37) is lower than an elastic modulus of the second adhesive (73).

6. The ultrasonic radiator (3) according to any one of Claims 1 to 5, wherein two adjacent lateral surfaces (11s) each being a lateral surface (11s) of a corresponding one of two adjacent elements of the plurality of plate-like elements (11) are bonded together with the first adhesive (37) .

7. The ultrasonic radiator (3) according to any one of Claims 1 to 6, wherein
the supporter (13) includes a partition portion (13f) that is located between two adjacent lateral surfaces (11s) each being a lateral surface (11s) of a corresponding one of two adjacent elements of the plurality of plate-like elements (11) and that is at least partially coated with the first adhesive (37), and
an elastic modulus of the supporter (13) is higher than an elastic modulus of the first adhesive (37).

8. The ultrasonic radiator (3) according to any one of Claims 1 to 7, further comprising a flexible substrate that is located opposite the radiation surface (11a) of at least one of the plurality of plate-like elements (11) with a space (71) between the flexible substrate and the at least one of the plurality of plate-like elements (11), wherein
the first adhesive (37) applied to the lateral surfaces (11s) of the at least one of the plurality of plate-like elements (11) extends off edges of the lateral surfaces (11s) to the flexible substrate and is applied to the flexible substrate to close at least part of a peripheral portion of the space (71).

9. The ultrasonic radiator (3) according to Claim 8, wherein
the supporter (13) includes a partition portion (13f) that is located between two adjacent lateral surfaces (11s), each being a lateral surface (11s) of a corresponding one of two adjacent elements of the plurality of plate-like elements (11), and that is at least partially coated with the first adhesive (37), and
an upper surface of the partition portion (13f) is located at a level above upper surfaces of the plurality of plate-like elements (11) and at a level below a lower surface of the flexible substrate.

10. The ultrasonic radiator (3) according to Claim 8 or 9, wherein
the at least one of the plurality of plate-like elements (11) is entirely covered with the first adhesive (37) from above the flexible substrate, and
the space (71) is hermetically sealed with the first adhesive (37).

11. The ultrasonic radiator (3) according to any one of Claims 8 to 10, further comprising a reinforcing member (75) that is located opposite the at least one of the plurality of plate-like elements (11) with the flexible substrate therebetween, the reinforcing member (75) extending over the flexible substrate and being at least partially coated with the first adhesive (37), wherein
an elastic modulus of the reinforcing member (75) is higher than an elastic modulus of the first adhesive (37).

12. The ultrasonic radiator (3) according to any one of Claims 1 to 11, wherein the plurality of plate-like elements (11) are each in a form of a flat plate.

13. An ultrasonic device (1), comprising:
the ultrasonic radiator (3) according to any one of Claims 1 to 12; and
a drive control unit (41) that supplies the plurality of plate-like elements (11) with alternating current of a frequency that falls within a frequency range of ultrasonic waves.

## Patentansprüche

1. Ultraschallstrahler (3), aufweisend:
eine Mehrzahl von plattenartigen Elementen (11), die jeweils auf einer Vorderseite oder einer Rückseite eine Strahlungsfläche (11a) aufweisen, von der aus Ultraschallwellen emittiert werden,
einen Träger (13), der die Mehrzahl von plattenartigen Elementen (11) in einer Anordnung hält, in der die Strahlungsflächen (11a) in unterschiedlichen Richtungsausrichtungen in Richtung zu dem gleichen Ort gerichtet sind, und
einen ersten Klebstoff (37), mit dem die Mehrzahl von plattenartigen Elementen (11) mit dem Träger (13) verbunden ist, wobei
die Mehrzahl von plattenartigen Elementen (11) jeweils eine Mehrzahl von Vibrationselementen (15) aufweist, die unterschiedlich in der Strahlungsfläche (11a) angeordnet sind und die Ultraschallwellen erzeugen,
wobei die Mehrzahl von plattenartigen Elementen (11) jeweils aufweist:
ein Elementsubstrat (19), das sich entlang der Strahlungsfläche (11a) erstreckt und die Mehrzahl von Vibrationselementen (15) aufweist, und
ein Hohlraumelement (21), das sich entlang der Strahlungsfläche (11a) erstreckt und eine Mehrzahl von Öffnungen (21c) aufweist, von denen jede mit einem korrespondierenden aus der Mehrzahl von Vibrationselementen (15) bedeckt ist,
wobei das Elementsubstrat (19) eine piezoelektrische Schicht (27) aufweist, die entlang der Strahlungsfläche (11a) Spannung erzeugt und durch Verbiegen und Verformen von Bereichen, die mit der Mehrzahl von Vibrationselementen (15) korrespondieren, schwingt,
wobei der Träger (13), das Hohlraumelement (21) und das Elementsubstrat (19) in dieser Reihenfolge in einer Richtung gestapelt sind, der die Strahlungsfläche (11a) zugewandt ist, und
der erste Klebstoff (37) auf seitliche Flächen (11s) von jedem aus der Mehrzahl von plattenartigen Elementen (11) und seitliche Flächen (21s) des Hohlraumelements (21) aufgetragen ist, die sich auf beiden Seiten mit der Strahlungsfläche (11a) dazwischen befinden.

2. Ultraschallstrahler (3) gemäß Anspruch 1, wobei der erste Klebstoff (37) auf die seitlichen Flächen (21s) des Hohlraumelements (21) und auf seitliche Flächen (19s) des Elementsubstrats (19), aufgetragen ist, die sich auf beiden Seiten mit der Strahlungsfläche (11a) dazwischen befinden.

3. Ultraschallstrahler (3) gemäß Anspruch 1 oder 2, wobei der Träger (13) periphere Abschnitte der Mehrzahl von plattenartigen Elementen (11) in einer Richtung überlappt, der die Strahlungsflächen (11a) der Mehrzahl von plattenartigen Elemente (11) zugewandt sind, wobei ein zweiter Klebstoff (73) zwischen dem Träger (13) und jedem der peripheren Abschnitte angeordnet ist.

4. Ultraschallstrahler (3) gemäß Anspruch 3, wobei ein Elastizitätsmodul des ersten Klebstoffs (37) höher als ein Elastizitätsmodul des zweiten Klebstoffs (73) ist.

5. Ultraschallstrahler (3) gemäß Anspruch 3, wobei ein Elastizitätsmodul des ersten Klebstoffs (37) kleiner als ein Elastizitätsmodul des zweiten Klebstoffs (73) ist.

6. Ultraschallstrahler (3) gemäß irgendeinem der Ansprüche 1 bis 5, wobei zwei benachbarte seitliche Flächen (11s), die jeweils eine seitliche Fläche (11s) eines korrespondierenden von zwei benachbarten Elementen der Mehrzahl von plattenartigen Elementen (11) sind, mit dem ersten Klebstoff (37) verbunden sind.

7. Ultraschallstrahler (3) gemäß irgendeinem der Ansprüche 1 bis 6, wobei
der Träger (13) einen Trennabschnitt (13f) aufweist, der zwischen zwei benachbarten seitlichen Flächen (11s) angeordnet ist, die jeweils eine seitlichen Fläche (11s) eines korrespondierenden von zwei benachbarten Elementen der Mehrzahl von plattenartigen Elementen (11) sind, und der zumindest teilweise mit dem ersten Klebstoff (37) beschichtet ist, und
ein Elastizitätsmodul des Trägers (13) höher als ein Elastizitätsmodul des ersten Klebstoffs (37) ist.

8. Ultraschallstrahler (3) gemäß irgendeinem der Ansprüche 1 bis 7, ferner aufweisend ein flexibles Substrat, das gegenüber der Strahlungsfläche (11a) von mindestens einem aus der Mehrzahl von plattenartigen Elementen (11) angeordnet ist, mit einem Raum (71) zwischen dem flexiblen Substrat und dem mindestens einen aus der Mehrzahl von plattenartigen Elementen (11), wobei
der erste Klebstoff (37), der auf die seitlichen Flächen (11s) des mindestens einen aus der Mehrzahl von plattenartigen Elementen (11) aufgebracht ist, sich von Rändern der seitlichen Flächen (11s) zu dem flexiblen Substrat erstreckt und auf das flexible Substrat aufgetragen wird, um mindestens einen Teil eines peripheren Abschnitts des Raums (71) zu schließen.

9. Ultraschallstrahler (3) gemäß Anspruch 8, wobei
der Träger (13) einen Trennabschnitt (13f) aufweist, der zwischen zwei benachbarten seitlichen Flächen (11s) angeordnet ist, wobei jede eine seitlichen Fläche (11s) eines korrespondierenden von zwei benachbarten Elementen der Mehrzahl von plattenartigen Elementen (11) ist, und der zumindest teilweise mit dem ersten Klebstoff (37) beschichtet ist, und
eine obere Fläche des Trennabschnitts (13f) auf einer Höhe über oberen Flächen der Mehrzahl von plattenartigen Elementen (11) und auf einer Höhe unter einer unteren Fläche des flexiblen Substrats angeordnet ist.

10. Ultraschallstrahler (3) gemäß Anspruch 8 oder 9, wobei
das mindestens eine aus der Mehrzahl von plattenartigen Elementen (11) vollständig mit dem ersten Klebstoff (37) von oberhalb des flexiblen Substrats bedeckt ist, und
der Raum (71) mit dem ersten Klebstoff (37) hermetisch abgedichtet ist.

11. Ultraschallstrahler (3) gemäß irgendeinem der Ansprüche 8 bis 10, ferner aufweisend ein Verstärkungselement (75), das gegenüber dem mindestens einen aus der Mehrzahl von plattenartigen Elementen (11) mit dem flexiblen Substrat dazwischen angeordnet ist, wobei sich das Verstärkungselement (75) über dem flexiblen Substrat erstreckt und zumindest teilweise mit dem ersten Klebstoff (37) beschichtet ist, wobei
ein Elastizitätsmodul des Verstärkungselements (75) höher als ein Elastizitätsmodul des ersten Klebstoffs (37) ist.

12. Ultraschallstrahler (3) gemäß irgendeinem der Ansprüche 1 bis 11, wobei die Mehrzahl von plattenartigen Elementen (11) jeweils in einer Form einer flachen Platte ist.

13. Ultraschallvorrichtung (1), aufweisend:
den Ultraschallstrahler (3) gemäß irgendeinem der Ansprüche 1 bis 12 und
eine Antriebssteuereinheit (41), die die Mehrzahl von plattenartigen Elementen (11) mit Wechselstrom einer Frequenz versorgt, die in einen Frequenzbereich von Ultraschallwellen fällt.

## Revendications

1. Radiateur à ultrasons (3), comprenant :
une pluralité d'éléments de type plaque (11) présentant chacun, sur une face avant ou une face arrière, une surface de rayonnement (11a) à partir de laquelle des ondes ultrasoniques sont émises ;
un support (13) qui maintient la pluralité d'éléments de type plaque (11) dans un arrangement dans lequel les surfaces de rayonnement respectives (11a) dans des orientations directionnelles différentes sont dirigées vers le même endroit ; et
un premier adhésif (37) avec lequel la pluralité d'éléments de type plaque (11) sont liés au support (13), dans lequel
la pluralité d'éléments de type plaque (11) comprennent chacun une pluralité d'éléments vibratoires (15) qui sont diversement situés dans la surface de rayonnement (11a) et qui génèrent des ondes ultrasoniques,
la pluralité d'éléments de type plaque (11) comprennent chacun :
un substrat d'élément (19) qui s'étend le long de la surface de rayonnement (11a) et comprend la pluralité d'éléments vibratoires (15), et
un élément de cavité (21) qui s'étend le long de la surface de rayonnement (11a) et présente une pluralité d'ouvertures (21c) dont chacune est recouverte d'un élément correspondant de la pluralité d'éléments vibratoires (15),
le substrat d'élément (19) comprend une couche piézoélectrique (27) qui produit une contrainte le long de la surface de rayonnement (11a) et vibre par des régions correspondant à la pluralité d'éléments vibratoires (15) étant pliés et déformés,
le support (13), l'élément de cavité (21) et le substrat d'élément (19) sont empilés dans cet ordre dans une direction à laquelle la surface de rayonnement (11a) fait face, et
le premier adhésif (37) est appliqué sur des surfaces latérales (11s) de chacun de la pluralité d'éléments de type plaque (11) et sur des surfaces latérales (21s) de l'élément de cavité (21) qui sont situées des deux côtés avec la surface de rayonnement (11a) entre elles.

2. Radiateur à ultrasons (3) selon la revendication 1, dans lequel le premier adhésif (37) est appliqué sur les surfaces latérales (21s) de l'élément de cavité (21) et sur des surfaces latérales (19s) du substrat d'élément (19) qui sont situées des deux côtés avec la surface de rayonnement (11a) entre elles.

3. Radiateur à ultrasons (3) selon la revendication 1 ou 2,
dans lequel le support (13) recouvre des parties périphériques de la pluralité d'éléments de type plaque (11) dans une direction à laquelle les surfaces de rayonnement (11a) de la pluralité d'éléments de type plaque (11) font face, un deuxième adhésif (73) étant situé entre le support (13) et chacune des parties périphériques.

4. Radiateur à ultrasons (3) selon la revendication 3, dans lequel un module d'élasticité du premier adhésif (37) est supérieur à un module d'élasticité du deuxième adhésif (73) .

5. Radiateur à ultrasons (3) selon la revendication 3, dans lequel un module d'élasticité du premier adhésif (37) est inférieur à un module d'élasticité du deuxième adhésif (73) .

6. Radiateur à ultrasons (3) selon l'une quelconque des revendications 1 à 5, dans lequel deux surfaces latérales adjacentes (11s), chacune étant une surface latérale (11s) d'un élément correspondant de deux éléments adjacents de la pluralité d'éléments de type plaque (11), sont liées ensemble avec le premier adhésif (37).

7. Radiateur à ultrasons (3) selon l'une quelconque des revendications 1 à 6, dans lequel
le support (13) comprend une partie de séparation (13f) qui est située entre deux surfaces latérales adjacentes (11s), chacune étant une surface latérale (11s) d'un élément correspondant de deux éléments adjacents de la pluralité d'éléments de type plaque (11), et qui est au moins partiellement recouverte du premier adhésif (37), et
un module d'élasticité du support (13) est supérieur à un module d'élasticité du premier adhésif (37).

8. Radiateur à ultrasons (3) selon l'une quelconque des revendications 1 à 7, comprenant en outre un substrat flexible situé à l'opposé de la surface de rayonnement (11a) d'au moins un de la pluralité d'éléments de type plaque (11), avec un espace (71) entre le substrat flexible et ledit au moins un de la pluralité d'éléments de type plaque (11), dans lequel
le premier adhésif (37) appliqué sur les surfaces latérales (11s) dudit au moins un de la pluralité d'éléments de type plaque (11) s'étend à partir de bords des surfaces latérales (11s) jusqu'au substrat flexible et est appliqué sur le substrat flexible pour fermer au moins une partie d'une partie périphérique de l'espace (71).

9. Radiateur à ultrasons (3) selon la revendication 8, dans lequel
le support (13) comprend une partie de séparation (13f) qui est située entre deux surfaces latérales adjacentes (11s), chacune étant une surface latérale (11s) d'un correspondant de deux éléments adjacents de la pluralité d'éléments de type plaque (11), et qui est au moins partiellement recouverte du premier adhésif (37), et
une surface supérieure de la partie de séparation (13f) est située à un niveau supérieur à des surfaces supérieures de la pluralité d'éléments de type plaque (11) et à un niveau inférieur à une surface inférieure du substrat flexible.

10. Radiateur à ultrasons (3) selon la revendication 8 ou 9, dans lequel
ledit au moins un de la pluralité d'éléments de type plaque (11) est entièrement recouvert du premier adhésif (37) depuis au-dessus du substrat flexible, et
l'espace (71) est hermétiquement fermé par le premier adhésif (37).

11. Radiateur à ultrasons (3) selon l'une quelconque des revendications 8 à 10, comprenant en outre un élément de renforcement (75) qui est situé à l'opposé dudit au moins un de la pluralité d'éléments de type plaque (11) avec le substrat flexible entre eux, l'élément de renforcement (75) s'étendant sur le substrat flexible et étant au moins partiellement recouvert du premier adhésif (37), dans lequel
un module d'élasticité de l'élément de renforcement (75) est supérieur à un module d'élasticité du premier adhésif (37).

12. Radiateur à ultrasons (3) selon l'une quelconque des revendications 1 à 11, dans lequel la pluralité d'éléments de type plaque (11) sont chacun sous une forme de plaque plate.

13. Dispositif à ultrasons (1), comprenant :
le radiateur à ultrasons (3) selon l'une quelconque des revendications 1 à 12 ; et
une unité de commande d'entraînement (41) qui alimente la pluralité d'éléments de type plaque (11) en courant alternatif d'une fréquence qui se situe dans une gamme de fréquences d'ondes ultrasoniques.
